# EUROPEAN PATENT SPECIFICATION

(11) **EP 4 168 106 B1**
(45) Date of publication and mention of the grant of the patent: **23.09.2026**
(21) Application number: 21829727.3
(22) Date of filing: 22.05.2021
(51) Int. Cl.: A61N 1/372, A61N 1/36, G06F 3/16, G16H 20/30, G16H 40/40, G16H 40/63, G16H 50/20, G16H 50/70

(54) **USER INTERFACE FOR PROSTHESIS**
BENUTZERSCHNITTSTELLE FÜR PROTHESE
INTERFACE UTILISATEUR POUR PROTHÈSE

(30) Priority: 22.06.2020 US 202063042089 P
(43) Date of publication of application: 26.04.2023
(73) Proprietor: Cochlear Limited, Macquarie University, New South Wales 2109 (AU)
(72) Inventor: STEFOPOULOS, Samuel, Macquarie University, New South Wales 2109 (AU); BOLNER, Federico, Macquarie University, New South Wales 2109 (AU); OPLINGER, Kenneth, Macquarie University, New South Wales 2109 (AU)
(74) Representative: Grünecker Patent- und Rechtsanwälte PartG mbB
(86) International application number: PCT/IB2021/054461
(87) International publication number: WO 2021/260457

(56) References cited:
- WO-A1-2015/024584
- WO-A1-2018/026435
- WO-A1-2019/193547
- US-A1- 2009 306 741
- US-A1- 2011 046 730
- US-A1- 2018 146 307
- US-A1- 2018 146 307
- US-B2- 9 854 370
- US-B2- 9 907 489

## Description

### BACKGROUND

US 2011/0046730 A1 discloses an at least partially implantable hearing prosthesis having a first mode of operation being a recharge mode of operation and a second mode of operation being a sound delivery mode of operation. In the recharge mode of operation, the prosthesis does not transmit microphone signals to the main implantable component. In the sound delivery mode of operation, the prosthesis is transferring processed microphone signals and power between an implantable microphone system and a main implantable component. The prosthesis may enter the recharge mode of operation and the sound delivery mode of operation based on user input.

Medical devices have provided a wide range of therapeutic benefits to recipients over recent decades. Medical devices can include internal or implantable components/devices, external or wearable components/devices, or combinations thereof (e.g., a device having an external component communicating with an implantable component). Medical devices, such as traditional hearing aids, partially or fully-implantable hearing prostheses (e.g., bone conduction devices, mechanical stimulators, cochlear implants, etc.), pacemakers, defibrillators, functional electrical stimulation devices, and other medical devices, have been successful in performing lifesaving and/or lifestyle enhancement functions and/or recipient monitoring for a number of years.

The types of medical devices and the ranges of functions performed thereby have increased over the years. For example, many medical devices, sometimes referred to as "implantable medical devices," now often include one or more instruments, apparatus, sensors, processors, controllers or other functional mechanical or electrical components that are permanently or temporarily implanted in a recipient. These functional devices are typically used to diagnose, prevent, monitor, treat, or manage a disease/injury or symptom thereof, or to investigate, replace or modify the anatomy or a physiological process. Many of these functional devices utilize power and/or data received from external devices that are part of, or operate in conjunction with, implantable components.

### SUMMARY

A device according to the invention includes the features defined in claim 1.

Embodiments include the features defined in the dependent claims.

### BRIEF DESCRIPTION OF THE DRAWINGS

Embodiments of the present invention are described below with reference to the attached drawings, in which:
FIG. 1 is a perspective view of an exemplary hearing prosthesis in which at least some of the teachings detailed herein are applicable;
FIG. 1A is a view of an exemplary sight prosthesis in which at least some of the teachings herein are applicable;
FIG. 2 schematically illustrates an implantable hearing system that incorporates an implantable microphone assembly and a motion sensor;
FIG. 3A functionally illustrates an exemplary use of adaptive filters;
FIG. 3B functionally depicts an exemplary embodiment of a system that is usable in the hearing prosthesis of FIG. 1 that functionally operates in accordance with the schematic of FIG. 3A;
FIG. 4 represents an exemplary flowchart according to an exemplary embodiment;
FIG. 5 pictorially represents some exemplary actions that can enable input that will be recognized by a prosthesis according to an exemplary embodiment;
FIG. 6 pictorially represents an exemplary scenario of use according to an exemplary embodiment;
FIG. 7 functionally illustrates an exemplary embodiment;
FIG. 8 functionally illustrates another exemplary embodiment; and
FIG. 9 presents an exemplary flowchart for an exemplary method.

### DETAILED DESCRIPTION

Merely for ease of description, the techniques presented herein are primarily described herein with reference to an illustrative medical device, namely a cochlear implant. However, it is to be appreciated that the techniques presented herein may also be used with a variety of other medical devices that, while providing a wide range of therapeutic benefits to recipients, patients, or other users, may benefit from the teachings herein used in other medical devices. For example, any techniques presented herein described for one type of hearing prosthesis, such as a cochlear implant, corresponds to a disclosure of another embodiment of using such teaching with another hearing prostheses, including bone conduction devices (percutaneous, active transcutaneous and/or passive transcutaneous), middle ear auditory prostheses, direct acoustic stimulators, and also utilizing such with other electrically simulating auditory prostheses (e.g., auditory brain stimulators), etc. The techniques presented herein can be used with implantable / implanted microphones, whether or not used as part of a hearing prosthesis (e.g., a body noise or other monitor, whether or not it is part of a hearing prosthesis). The techniques presented herein can also be used with vestibular devices (e.g., vestibular implants), sensors, seizure devices (e.g., devices for monitoring and/or treating epileptic events, where applicable), sleep apnea devices, electroporation, etc., and thus any disclosure herein is a disclosure of utilizing such devices with the teachings herein, providing that the art enables such. It is also noted that in an exemplary embodiment, the teachings herein can be used with a retinal implant device. Thus, any disclosure herein corresponds to a disclosure of expanding functionality to include the functionality of a retinal implant, and, for example, any disclosure of a cochlear implant processor corresponds to a light processor. In further embodiments, the techniques presented herein may be used with air purifiers or air sensors (e.g., automatically adjust depending on environment), hospital beds, identification (ID) badges/bands, or other hospital equipment or instruments, where such rely upon behind the ear devices.

By way of example, any of the technologies detailed herein which are associated with components that are implanted in a recipient can be combined with information delivery technologies disclosed herein, such as for example, devices that evoke a hearing percept and/or devices that evoke a vision percept, to convey information to the recipient. By way of example only and not by way of limitation, a sleep apnea implanted device can be combined with a device that can evoke a hearing percept so as to provide information to a recipient, such as status information, etc. In this regard, the various sensors detailed herein and the various output devices detailed herein can be combined with such a non-sensory prosthesis or any other nonsensory prosthesis that includes implantable components so as to enable a user interface as will be described herein that enables information to be conveyed to the recipient, which information is associated with the implant.

Moreover, embodiments need not necessarily provide input or status information to the recipient. Instead, the various sensors detailed herein can be utilized in combination with the nonsensory implants detailed herein so as to enable control or performance adjustments of the implanted component. For example, the embodiments that utilize sensors and the associated logic circuitry that would be combined with a sleep apnea device, for example, can be utilized to enable the recipient to input commands to control the implant. Such can potentially also be done with respect to a bionic arm or bionic leg, etc. In this regard, embodiments can enable a user interface that can enable a recipient to provide input into the prosthesis to control the prosthesis without utilizing any artificial external component. For example, embodiments can enable the input utilizing only the recipient's voice and/or only the recipient's hand / fingers. Thus, embodiments can enable control of such prostheses utilizing only a recipient's hand and/or only a recipient's voice. Accordingly, at least some exemplary embodiments can combine hearing prosthesis technology with the innovations detailed herein with other implant technologies to enable control without the need of other artificial devices.

Thus, the teachings detailed herein are implemented in sensory prostheses, such as hearing devices, including hearing implants specifically, and neural stimulation devices in general. Other types of sensory prostheses can include retinal implants. Accordingly, any teaching herein with respect to a sensory prosthesis corresponds to a disclosure of utilizing those teachings in / with a hearing implant and in / with a retinal implant, unless otherwise specified, providing the art enables such. To be clear, any teaching herein with respect to a specific sensory prosthesis corresponds to a disclosure of utilizing those teachings in / with any of the aforementioned hearing prostheses, and *vice versa.* Corollary to this is at least some teachings detailed herein can be implemented in somatosensory implants and/or chemosensory implants. Accordingly, any teaching herein with respect to a sensory prosthesis corresponds to a disclosure of utilizing those teachings with/in a somatosensory implant and/or a chemosensory implant.

While the teachings detailed herein will be described for the most part with respect to hearing prostheses, in keeping with the above, it is noted that any disclosure herein with respect to a hearing prosthesis corresponds to a disclosure of another embodiment of utilizing the associated teachings with respect to any of the other prostheses noted herein, whether a species of a hearing prosthesis, or a species of a sensory prosthesis, such as a retinal prosthesis. In this regard, any disclosure herein with respect to evoking a hearing percept corresponds to a disclosure of evoking other types of neural percepts in other embodiments, such as a visual / sight percept, a tactile percept, a smell precept or a taste percept, unless otherwise indicated and/or unless the art does not enable such. Any disclosure herein of a device, system, and/or method that is used to or results in ultimate stimulation of the auditory nerve corresponds to a disclosure of an analogous stimulation of the optic nerve utilizing analogous components / methods / systems. All of this can be separately or in combination.

Embodiments detailed herein focus on the utilization of a hearing prosthesis to provide status and information a recipient. It is to be understood that in some embodiments, a retinal prosthesis can be utilized to provide visual input to the recipient. By way of example only and not by way of limitation, in an exemplary embodiment, the retinal prosthesis can be configured to results in a vision of an artificial image, which can correspond to words or the like, which can correspond to a status of the prostheses. Accordingly, any disclosure herein associated with providing sound-based or hearing percept base information the recipient also corresponds to a disclosure of providing vision based information to the recipient and *vice versa.*

Moreover, the teachings detailed herein utilizing touch sensors and/or boy sensors can be utilized with a retinal prosthesis. In this regard, consistent with the theme detailed above, in some exemplary embodiments, technologies associated with hearing prostheses, such as a microphone and/or an accelerometer, can be combined with the teachings detailed herein and a retinal prostheses to enable control of the retinal prostheses in a manner analogous to enabling control of the hearing prostheses as detailed below.

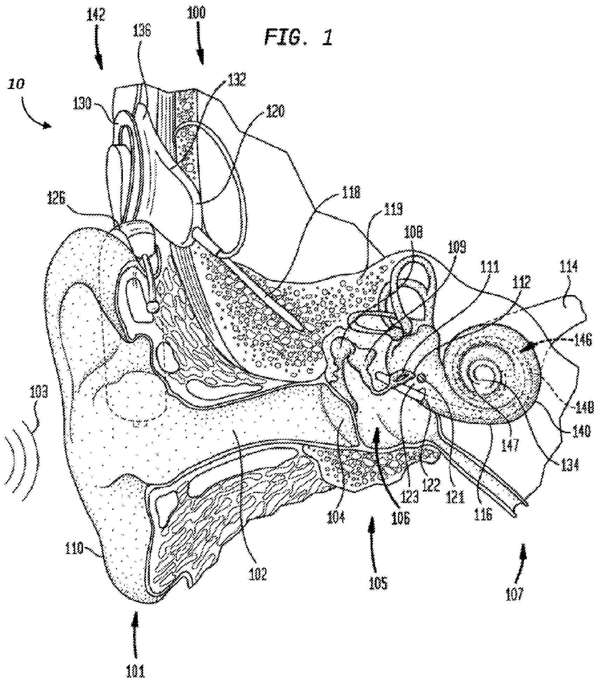

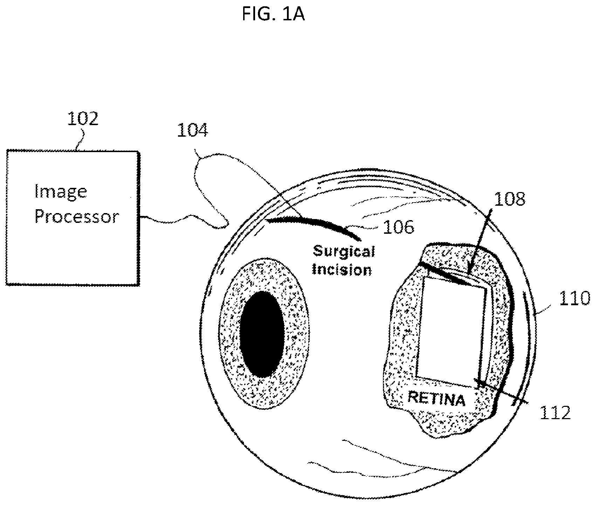

FIG. 1 is a perspective view of a totally implantable cochlear implant, referred to as cochlear implant 100, implanted in a recipient, to which some embodiments detailed herein and/or variations thereof are applicable. The totally implantable cochlear implant 100 is part of a system 10 that can include external components, in some embodiments, as will be detailed below. It is noted that the teachings detailed herein are applicable, in at least some embodiments, to any type of hearing prosthesis having an implantable microphone. The teachings detailed herein are also applicable, in at least some embodiments, to any type of hearing prosthesis not having an implantable microphone, and thus are applicable to non-totally implantable hearing prostheses.

It is noted that in alternate embodiments, the teachings detailed herein and/or variations thereof can be applicable to other types of hearing prostheses, such as, for example, bone conduction devices (e.g., active transcutaneous bone conduction devices), Direct Acoustic Cochlear Implant (DACI), etc. Embodiments can include any type of hearing prosthesis that can utilize the teachings detailed herein and/or variations thereof. It is further noted that in some embodiments, the teachings detailed herein and/or variations thereof can be utilized by other types of prostheses beyond hearing prostheses.

The recipient has an outer ear 101, a middle ear 105, and an inner ear 107. Components of outer ear 101, middle ear 105, and inner ear 107 are described below, followed by a description of cochlear implant 100.

In a fully functional ear, outer ear 101 comprises an auricle 110 and an ear canal 102. An acoustic pressure or sound wave 103 is collected by auricle 110 and channeled into and through ear canal 102. Disposed across the distal end of ear channel 102 is a tympanic membrane 104 which vibrates in response to sound wave 103. This vibration is coupled to oval window or fenestra ovalis 112 through three bones of middle ear 105, collectively referred to as the ossicles 106 and comprising the malleus 108, the incus 109, and the stapes 111. Bones 108, 109, and 111 of middle ear 105 serve to filter and amplify sound wave 103, causing oval window 112 to articulate, or vibrate in response to vibration of tympanic membrane 104. This vibration sets up waves of fluid motion of the perilymph within cochlea 140. Such fluid motion, in turn, activates tiny hair cells (not shown) inside of cochlea 140. Activation of the hair cells causes appropriate nerve impulses to be generated and transferred through the spiral ganglion cells (not shown) and auditory nerve 114 to the brain (not shown) where they are perceived as sound.

As shown, cochlear implant 100 comprises one or more components which are temporarily or permanently implanted in the recipient. Cochlear implant 100 is shown in FIG. 1 with an external device 142, that is part of system 10 (along with cochlear implant 100), which, as described below, is configured to provide power to the cochlear implant, where the implanted cochlear implant includes a battery that is recharged by the power provided from the external device 142. In the illustrative arrangement of FIG. 1, external device 142 can comprise a power source (not shown) disposed in a Behind-The-Ear (BTE) unit 126. External device 142 also includes components of a transcutaneous energy transfer link, referred to as an external energy transfer assembly. The transcutaneous energy transfer link is used to transfer power and/or data to cochlear implant 100. Various types of energy transfer, such as infrared (IR), electromagnetic, capacitive and inductive transfer, may be used to transfer the power and/or data from external device 142 to cochlear implant 100. In the illustrative embodiments of FIG. 1, the external energy transfer assembly comprises an external coil 130 that forms part of an inductive radio frequency (RF) communication link. External coil 130 is typically a wire antenna coil comprised of multiple turns of electrically insulated single-strand or multi-strand platinum or gold wire. External device 142 also includes a magnet (not shown) positioned within the turns of wire of external coil 130. It should be appreciated that the external device shown in FIG. 1 is merely illustrative, and other external devices may be used with embodiments of the present invention.

Cochlear implant 100 comprises an internal energy transfer assembly 132 which can be positioned in a recess of the temporal bone adjacent auricle 110 of the recipient. As detailed below, internal energy transfer assembly 132 is a component of the transcutaneous energy transfer link and receives power and/or data from external device 142. In the illustrative embodiment, the energy transfer link comprises an inductive RF link, and internal energy transfer assembly 132 comprises a primary internal coil 136. Internal coil 136 is typically a wire antenna coil comprised of multiple turns of electrically insulated single-strand or multi-strand platinum or gold wire.

Cochlear implant 100 further comprises a main implantable component 120 and an elongate electrode assembly 118. In some embodiments, internal energy transfer assembly 132 and main implantable component 120 are hermetically sealed within a biocompatible housing. In some embodiments, main implantable component 120 includes an implantable microphone assembly (not shown) and a sound processing unit (not shown) to convert the sound signals received by the implantable microphone in internal energy transfer assembly 132 to data signals. That said, in some alternative embodiments, the implantable microphone assembly can be located in a separate implantable component (e.g., that has its own housing assembly, etc.) that is in signal communication with the main implantable component 120 (e.g., via leads or the like between the separate implantable component and the main implantable component 120). In at least some embodiments, the teachings detailed herein and/or variations thereof can be utilized with any type of implantable microphone arrangement. Some additional details associated with the implantable microphone assembly 137 will be detailed below.

Main implantable component 120 further includes a stimulator unit (not shown) which generates electrical stimulation signals based on the data signals. The electrical stimulation signals are delivered to the recipient via elongate electrode assembly 118.

Elongate electrode assembly 118 has a proximal end connected to main implantable component 120, and a distal end implanted in cochlea 140. Electrode assembly 118 extends from main implantable component 120 to cochlea 140 through mastoid bone 119. In some embodiments, electrode assembly 118 may be implanted at least in basal region 116, and sometimes further. For example, electrode assembly 118 may extend towards apical end of cochlea 140, referred to as cochlea apex 134. In certain circumstances, electrode assembly 118 may be inserted into cochlea 140 via a cochleostomy 122. In other circumstances, a cochleostomy may be formed through round window 121, oval window 112, the promontory 123 or through an apical turn 147 of cochlea 140.

Electrode assembly 118 comprises a longitudinally aligned and distally extending array 146 of electrodes 148, disposed along a length thereof. As noted, a stimulator unit generates stimulation signals which are applied by electrodes 148 to cochlea 140, thereby stimulating auditory nerve 114.

As noted above, cochlear implant 100 comprises a totally implantable prosthesis that is capable of operating, at least for a period of time, without the need for external device 142. Therefore, cochlear implant 100 can further comprise a rechargeable power source (not shown) that stores power received from external device 142. The power source can comprise, for example, a rechargeable battery. During operation of cochlear implant 100, the power stored by the power source is distributed to the various other implanted components as needed. The power source may be located in main implantable component 120, or disposed in a separate implanted location.

It is noted that the teachings detailed herein and/or variations thereof can be utilized with a non-totally implantable prosthesis. That is, in an alternate embodiment of the cochlear implant 100, the cochlear implant 100 is a traditional hearing prosthesis.

In some exemplary embodiments, a signal sent to the stimulator of the cochlear implant can be derived from an external microphone, in which case the system is called a semi-implantable device, or from an implanted microphone, which then refers to a fully implantable device. DACIs and other types of implants can also use an implanted microphone, and thus are also fully implantable devices. Fully implantable devices can have utility by presenting improved cosmesis, can have an improved immunity to certain noises (e.g., wind noise), can present few opportunities for loss or damage, and can at least sometimes be more resistant to clogging by debris or water, etc. DACIs can have utilitarian value by keeping the ear canal open, which can reduce the possibility of infection of the ear canal, which otherwise is humid, often impacted with cerumen (earwax), and irritated by the required tight fit of a non-implanted hearing aid.

FIG. 1A presents an exemplary embodiment of a neural prosthesis in general, and a retinal prosthesis and an environment of use thereof, in particular. In some embodiments of a retinal prosthesis, a retinal prosthesis sensor-stimulator 108 is positioned proximate the retina 110. In an exemplary embodiment, photons entering the eye are absorbed by a microelectronic array of the sensor-stimulator 108 that is hybridized to a glass piece 112 containing, for example, an embedded array of microwires. The glass can have a curved surface that conforms to the inner radius of the retina. The sensor-stimulator 108 can include a microelectronic imaging device that can be made of thin silicon containing integrated circuitry that convert the incident photons to an electronic charge.

An image processor 102 is in signal communication with the sensor-stimulator 108 via cable 104 which extends through surgical incision 106 through the eye wall (although in other embodiments, the image processor 102 is in wireless communication with the sensor-stimulator 108). In an exemplary embodiment, the image processor 102 is analogous to the sound processor / signal processors of the auditory prostheses detailed herein, and in this regard, any disclosure of the latter herein corresponds to a disclosure of the former in an alternate embodiment. The image processor 102 processes the input into the sensor-stimulator 108, and provides control signals back to the sensor-stimulator 108 so the device can provide processed and output to the optic nerve. That said, in an alternate embodiment, the processing is executed by a component proximate to or integrated with the sensor-stimulator 108. The electric charge resulting from the conversion of the incident photons is converted to a proportional amount of electronic current which is input to a nearby retinal cell layer. The cells fire and a signal is sent to the optic nerve, thus inducing a sight perception.

The retinal prosthesis can include an external device disposed in a Behind-The-Ear (BTE) unit or in a pair of eyeglasses, or any other type of component that can have utilitarian value. The retinal prosthesis can include an external light / image capture device (e.g., located in / on a BTE device or a pair of glasses, etc.), while, as noted above, in some embodiments, the sensor-stimulator 108 captures light / images, which sensor-stimulator is implanted in the recipient.

In the interests of compact disclosure, any disclosure herein of a microphone or sound capture device corresponds to an analogous disclosure of a light / image capture device, such as a charge-coupled device. Corollary to this is that any disclosure herein of a stimulator unit which generates electrical stimulation signals or otherwise imparts energy to tissue to evoke a hearing percept corresponds to an analogous disclosure of a stimulator device for a retinal prosthesis. Any disclosure herein of a sound processor or processing of captured sounds or the like corresponds to an analogous disclosure of a light processor / image processor that has analogous functionality for a retinal prosthesis, and the processing of captured images in an analogous manner. Indeed, any disclosure herein of a device for a hearing prosthesis corresponds to a disclosure of a device for a retinal prosthesis having analogous functionality for a retinal prosthesis. Any disclosure herein of fitting a hearing prosthesis corresponds to a disclosure of fitting a retinal prosthesis using analogous actions. Any disclosure herein of a method of using or operating or otherwise working with a hearing prosthesis herein corresponds to a disclosure of using or operating or otherwise working with a retinal prosthesis in an analogous manner.

Implanted microphones can detect pressure. In at least some embodiments, implanted microphones are configured to detect air pressure, which is subsequently transmitted through the tissue to the microphone, such as to a diaphragm or membrane of the microphone. Implanted microphones can detect other pressures presented to their surface, which can be undesirable in certain circumstances. One type of pressure which can represent an impairment to the performance of an implanted microphone is pressure due to acceleration. In some embodiments, such acceleration can have a deleterious effect on a hearing prosthesis if it is in the desired operational frequency range of the prosthesis, typically 20 Hz to 20 kHz, although narrower ranges still give satisfactory speech intelligibility. Accelerations may arise from, for example, foot impact during walking, motion of soft tissue relative harder tissues, wear of harder tissues against each other, chewing, blowing one's nose, a grumbling stomach, laughing, and vocalization, etc.

In some embodiments, the accelerations induce pressure on the microphone, which cannot distinguish the desired pressure due to external sounds from what may be undesirable pressure due to internal vibration originating directly from the body. The accelerations can be thought of as giving rise to these pressures by virtue of the microphone being driven into the tissue. If the microphone is securely mounted on the skull, and the skull vibrates normal to its surface, the microphone diaphragm will be driven into the tissue which, due to the mass, and hence inertia of the tissue, can present a reactive force to the microphone. That reactive force divided by the area of the microphone is the pressure generated by acceleration.

In some instances, there can be utilitarian value to reducing signal outputs due to acceleration. Because the relative body-borne to air-borne pressure of an implanted microphone is typically 10-20 dB higher than that that occurs in normal hearing, body originating sounds can be louder relative to externally originating sound. Such large ratios of vibration to acoustic signals are experienced by a recipient as banging and crashing during movement, very noisy chewing, and their own voice being abnormally loud relative to other speakers. At the same time, it is noted that there is utilitarian value in some instances in avoiding the cancellation of all or part of the recipient's own voice. Complete cancellation of the recipient's own voice can result in, in some scenarios, the recipient speaking very loudly compared to other speakers. It is therefore utilitarian to reduce the ratio of vibration to acoustic signals to a level, such as a comparable level, to that found in normal hearing.

An exemplary embodiment that includes an implantable microphone assembly utilizes a motion sensor to reduce the effects of noise, including biological noise, in an output response of the implantable microphone assembly. In an exemplary embodiment, the diaphragm of the implantable microphone assembly that vibrates as a result of waves traveling through the skin of the recipient originating from an ambient sound, can be also affected by body noise and the like. To actively address non-ambient noise sources (e.g., body noise conducted through tissue of a recipient to a microphone, which in at least some embodiments is not of an energy level and/or frequency to be audible at a location away from the recipient, at least not without sound enhancement devices) of vibration of the diaphragm of the implantable microphone and thus the resulting undesired movement between the diaphragm and overlying tissue, some embodiments utilize a motion sensor to provide an output response proportional to the vibrational movement experienced by the microphone assembly. Generally, the motion sensor can be mounted anywhere such that it enables the provision of a sufficiently accurate representation of the vibration received by the implantable microphone in general, and the diaphragm of the implantable microphone in particular. The motion sensor can be part of the assembly that contains the microphone/diaphragm thereof, while in an alternate embodiment, it can be located in a separate assembly (e.g. a separate housing, etc.). In an exemplary embodiment, the motion sensor is at least partially isolated from the receipt of the ambient acoustic signals originating from an ambient sound that pass transcutaneously through the tissue located over the microphone/diaphragm of the microphone and which are received by the microphone diaphragm. In this regard, the motion sensor can provide an output response/signal that is indicative of motion (e.g., caused by vibration and/or acceleration), whereas a transducer of the microphone can generate an output response/signal that is indicative of both transcutaneously received acoustic sound and motion. Accordingly, the output response of the motion sensor can be removed from the output response of the microphone to reduce the effects of motion on the implanted hearing system.

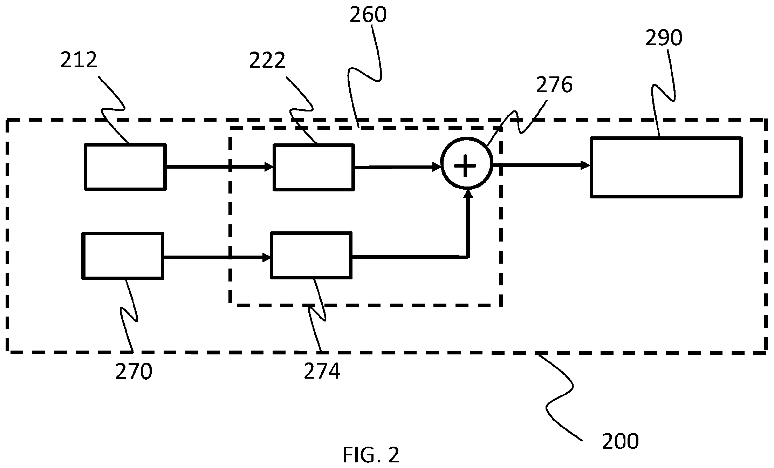

Accordingly, to remove noise, including biological noise (and in some instances, feedback), it is utilitarian to measure the acceleration of the microphone assembly. FIG. 2 schematically illustrates an implantable hearing system that incorporates an implantable component of a hearing prosthesis 200 including a microphone assembly having a microphone 212 and motion sensor 270 (which in some embodiments can be an accelerometer). As shown, the motion sensor 270 further includes a filter 274 (although in other embodiments, filter 274 is not present) that is utilized for matching the output response of the motion sensor 270 to the output response of the microphone 212 (where the output thereof is also filtered, in some embodiments, by filter 222). One or both of the filters 222 and 274 can be adaptive filters that adapt to changing circumstances. Of note, the diaphragm of microphone 212 is subject to desired acoustic signals (i.e., from an ambient source 103), as well as undesired signals from biological sources (e.g., vibration caused by talking, chewing, etc.) and, depending on the type of output device 290 (e.g., bone conduction vibratory apparatus, DACI actuator, middle ear actuator (embodiments can be used with middle ear implants), etc.) feedback from the output device 290 received by a tissue feedback loop extending from the output device 290 to the microphone 212 and the motion sensor 270. In contrast, the motion sensor 270 is at least partially isolated (which includes totally isolated) from the ambient source and is subjected to the undesired signals caused by the biological source and/or by feedback received via the feedback loop. Accordingly, the output of the motion sensor 270 corresponds to some or all of the undesired signal components of the microphone 212. However, the magnitude of the output channels (i.e., the output response of the microphone 212 and output response of the motion sensor 270) can be different and/or shifted in phase. In order to remove the undesired signal components from the microphone output response, the filter 274 and/or the system processor can be operative to filter one or both of the responses to provide scaling, phase shifting and/or frequency shaping. The output responses of the microphone 212 and motion sensor 270 are then combined by summation unit 276, which generates a net output response that has a reduced response to the undesired signals, which net output response is used to operate the output device 290.

Collectively, filters 222 and 274, the adder 276, and any control equipment used to control these components correspond a noise cancellation sub-system 260.

In order to implement a filter 274 for scaling and/or phase shifting the output response of a motion sensor 270 to remove the effects of feedback and/or biological noise from a microphone 212 output response, a system model of the relationship between the output responses of the microphone 212 and motion sensor 270 is identified/developed.

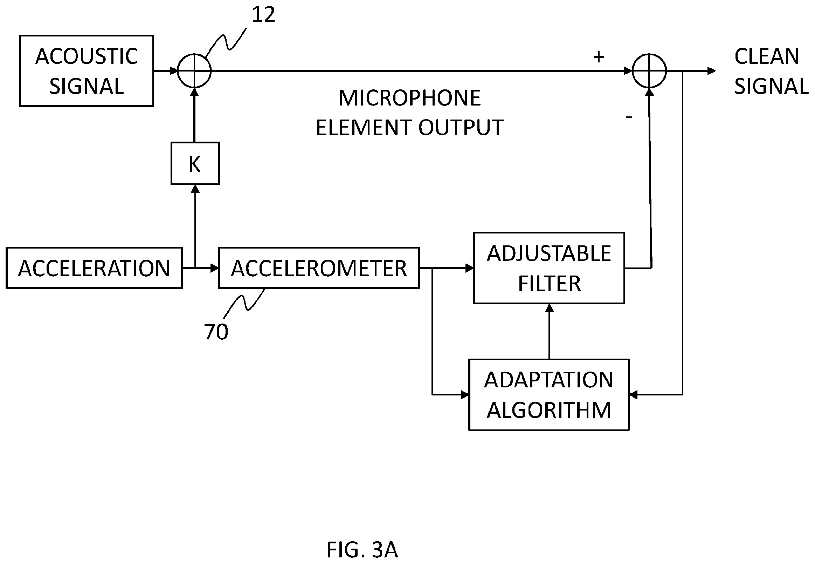

As noted above, an exemplary embodiment utilizes adaptive filter(s) to filter out body noise and the like. More particularly, FIG. 3A functionally illustrates an exemplary use of such adaptive filters. In FIG. 3A, biological noise is modeled by the acceleration at the microphone assembly filtered through a linear process K. This signal is added to the acoustic signal at the surface of the microphone element. In this regard, the microphone 212 sums the signals. If the combination of K and the acceleration are known, the combination of the accelerometer output and the adaptive/adjustable filter can be adjusted to be K. This is then subtracted out of the microphone output. This will result in the cleansed or net audio signal with a reduced biological noise component. This net signal may then be passed to the signal processor where it can be processed by the hearing system.

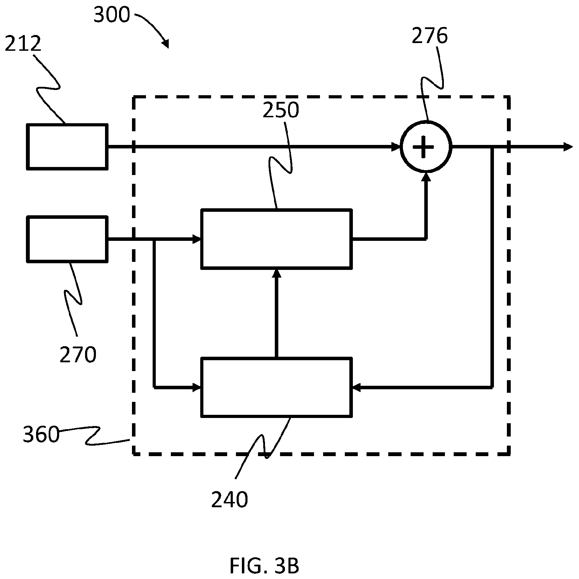

FIG. 3B functionally depicts an exemplary embodiment of a system 300 that is usable in the hearing prosthesis 10 of FIG. 1 and that functionally operates in accordance with the schematic of FIG. 3A. The system 300 includes microphone 212 and accelerometer 270. The microphone 212 is configured such that it receives signals resulting from the ambient sound, as well as biological noise/body noise, including, in at least some embodiments, signals resulting from a recipient's own voice that travel through the body via bone conduction/tissue conduction, and other own body conducted noises (e.g., noises originating from coughing, blowing one's nose, etc.). These latter signals are added at the microphone 212 to the signals resulting from ambient sound, because the microphone 212 detects both signals. Conversely, accelerometer 270 is at least partially isolated from the signals resulting from the ambient sound, and generally responds to body noise signals and/or feedback signals. The system 300 incorporates an adjustable filter apparatus 250 controlled by a control unit 240 that runs an adaptive algorithm to control the filter(s) of the adjustable filter apparatus 250. Briefly, as can be seen, the output of the adaptive filter apparatus 250, controlled by filter control unit 240, is fed to adder 276, wherein it is added to (or, more accurately, subtracted from) the output of the microphone 212, and passed on to a signal processor and/or an output device (not shown, but for example, a receiver stimulator of a cochlear implant, an actuator of a DACI, and/or an actuator (vibrator) of an active transcutaneous bone conduction device) of the system. Collectively, the adjustable filters 250, the filter control unit 240, and the adder 276 correspond to an adaptive noise cancellation sub-system 360, which can include additional components (e.g., filters in between the microphone 212 and the mixer / adder 276).

The functionality and operation of the adaptive noise cancellation sub-system 360 can be more detailed / involved than that just described. Some exemplary embodiments can utilize very advanced adaptive noise cancellation techniques. That said, in alternative embodiments, as inferred above, some systems do not utilize an adaptive noise cancellation system, and can instead utilize traditional systems. Any device, system, and/or method that can enable noise cancellation sub-system 360 to operate can be utilized in at least some embodiments.

Teachings herein, in some instances, combine one or more of the above features in a prosthesis that includes the functionality of a wakeable user interface that can enable the user to control functionalities (e.g., change volume/program) and/or retrieve status information (e.g., battery percentage or program information / status) of their device. Some embodiments rely on the above one or more features to enable the wakeable user interface and/or otherwise provide a more utilitarian makeable interface relative to that which would otherwise be the case. As will be detailed below, embodiments can include a smartphone application that can be used for user training and to calibrate the algorithm(s) employed to recognize input. However, to be clear, embodiments can include operating the wakeable user interface and inputting input therein without an artificial device outside the body. That is, embodiments are directed to a wakeable user interface that enables input solely by touch and/or voice and/or any other abilities that humans have as a result of being human. In some embodiments, the user interface, once entered from a regular mode, using a predetermined input / after receiving a pre-determined input by the prosthesis, can be controlled / activated via commands. For example, the prosthesis can be configured to recognize sequences of pre-defined events (such as taps/swipes on the skin overlying a sensor or other input receiver) or voice commands and respond accordingly (such pre-defined events can also initiate the user interface mode). In this regard, a predetermined pattern and/or utterance can correspond to a wake command, and the prosthesis can be configured to recognize such as an alert to the device that an incoming action command is about to be provided by the user. Then, the device can receive the incoming action command and respond accordingly.

Conversely, the prosthesis can be configured to ignore an action command if not in the user interface / if the prosthesis did not recognize the alert / did not receive the alert. By rough analogy, this is like pushing a gas pedal in a car where the key has not yet been turned. This can reduce the likelihood of so-called false positives.

More specifically, in an exemplary embodiment, the prosthesis is configured so as to operate in a regular mode where the prosthesis operates normally and ignores all commands except for the wake command. The prosthesis can be further configured such that when the prosthesis recognizes the wake command, the prosthesis enters the user interface mode (as distinguished from the regular mode). There can be utilitarian value with respect to this regime as compared to the "always on" concept where the recipient will react to or otherwise respond to the various commands detailed herein that are originated via touch and/or via voice in that such can, in at least some embodiments, minimize the occurrence of false positives relative to that which would otherwise be the case. In this regard, as used herein, false positives correspond to occurrences that are incorrectly identified as commands and which should have been instead ignored and/or resulted in the prosthesis operating in a manner differently (where it should not have operated in a manner differently) than that which was the case prior to the false positive. Note that this is different than the prosthesis merely interpreting a command incorrectly relative to another command. A false positive is the prosthesis interpreting input as a command when the input was not a command.

Briefly, in an exemplary embodiment with respect to regular mode, the prosthesis can capture sound, such as capturing sound which results from sound impinging upon the outer skin of a recipient and being transmitted through the recipient to the implanted microphone / subcutaneous microphone, where the microphone transduces the impinging energy into an electrical output signal which is provided to an implanted sound processor or to another set of electronics that utilizes this signal to stimulate tissue of the recipient via an implanted stimulator to results in a hearing percept. This is regular operation of a prosthesis. Regular operation of the prosthesis also includes utilizing noise cancellation techniques and/or feedback mitigation techniques. Regular operation of the prosthesis also includes scene classification in some embodiments and/or power saving routines. Moreover, in at least some exemplary embodiments, a regular mode can be a mode in which unprompted indicators are provided by the prosthesis to the recipient, such as automatically indicating a low battery state or the like.

Regular operation of a prosthesis also includes a sleep mode where the prosthesis is not being utilized or otherwise is in a dormant state. This is distinguished from a user interface mode as will be detailed herein. Indeed, as used herein, the phrase wake command herein is not to be confused with a wake command that simply transitions the prosthesis from a sleep mode to a non-sleep mode. The wake commands detailed herein transition the prosthesis from a mode where the user interface regime is not operational or otherwise the prosthesis will not respond to input commands that are inputted in accordance with the teachings herein via touch and/or via voice to a mode where the user interface regime is operational or otherwise the prosthesis will respond to input commands. By way of example only and not by way of limitation, a command that would be responded to when the prosthesis is in the user interface mode would not be responded to when the prosthesis is not in the user interface mode or otherwise when the prosthesis is in the regular use state. Accordingly, another way of explaining the difference between the regular mode and the user interface mode (or regular state versus the user interface state as sometimes referred to herein) is that one or more or all of the commands that would be responded to when in the user interface mode will not be responded to when in the regular mode. Another way of explaining the difference in the two modes is that the only tactile and/or voice input command that will be responded to when in the regular mode is the command to enter the user interface mode (herein referred to as the "wake command").

In at least some exemplary embodiments, the prosthesis is configured such that when in the user interface mode, after entering from the regular mode, the user / recipient can input action commands so as to control functions of the prostheses via the aforementioned tactile and/or voice input, etc., and via the regimes that will be detailed below. In an exemplary embodiment, control functions can correspond to a change in volume and/or muting of the output of the prosthesis. Thus, the input of the action command to do a change volume control function could raise or lower the volume, depending on the action command. A control function can correspond to a change between external hearing (i.e.., utilizing an external device that utilizes a sound capture apparatus or a sound input apparatus) and the so-called invisible hearing (i.e., utilizing only the implanted microphone of the hearing prostheses) without an external component. A control function can also correspond to changing an invisible hearing program and/or activating or deactivating a "sleep mode." With regard to the latter, while the prosthesis can enter the sleep mode in the regular mode of operation, either manually or as a result of control by the recipient (e.g., by utilizing an external component to provide input to the implanted device, such as by a inductance communication system in a traditional manner), the sleep mode can be entered as a result of commands received during the user interface mode of operation, the sleep mode being entered as a result of tactile and/or voice commands as opposed to the utilization of an external device utilized during the regular mode. Additional control functions can correspond to pause/play of recorded material and/or the activation and/or deactivation and/or control of aggressiveness of a noise reduction algorithm. Any control function that can be enabled by the teachings herein can be applicable, providing that the art enables such and there is utilitarian value therefore.

It is also noted that in at least some exemplary embodiments, even while in the user interface mode, an external device or the like can be utilized to control or otherwise adjust the operation of the implanted prosthesis. Indeed, in some exemplary embodiments, during the normal mode of operation, an external device or the like can be utilized to control the normal mode of operation. In an exemplary embodiment, one or more all of the things the prosthesis will do or otherwise how the prosthesis will operate when in the user interface mode can be entered when in the regular mode utilizing an external device. For example, if the user interface mode permits volume control, so can such be done in the regular mode. If the user interface mode permits change of programming, so to can such be done in the regular mode. In an exemplary embodiment, the external device can be a device that requires close proximity to the implanted component, such as that which is associated with an inductance communication coil, where the external device can be utilized to input commands or otherwise provide input to the implanted device utilizing an inductance coil, which inductance coil can in at least some exemplary embodiments also be utilized to charge an implanted battery or other power storage device to enable the implant to operate without an external power source. In an exemplary embodiment, radiofrequency technology, such as for example, Bluetooth technology, can be utilized to communicate with a handheld or body carry device that is more than 2, 3, 4, 5, 6, 7, 8, 9, or 10 centimeters away from the implanted component so as to adjust the prostheses when in the regular mode of operation (or when in the user interface mode of operation). In an exemplary embodiment the handheld and/or body worn and/or body carried device can be a smart phone or a smart watch or a remote hearing assistant device that is a specialized device used specifically to control the implanted prostheses.

In some embodiments, the prosthesis can be configured such that the inputted action commands can result in the retrieval of status information about the device, such as for example only and not by way of limitation, battery charge percentage and/or amounts, programming number or identifier, data associated with therapy and/or adjustment of the device, such as the date and/or time of the next scheduled visit to a hearing prostheses clinic, etc.

Also, in at least some embodiments, the prosthesis can be configured such that the inputted action commands can result in the prosthesis exiting the user interface mode and returning to the regular mode of operation.

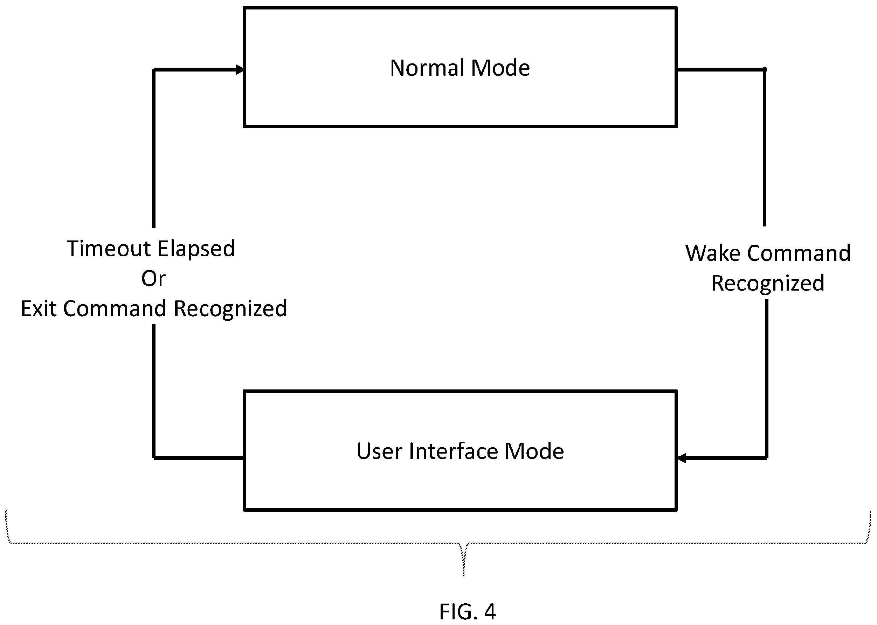

FIG. 4 presents a simplified exemplary flowchart for an exemplary algorithm that can be utilized by an exemplary prosthesis according to an exemplary embodiment. In this regard, the exemplary prosthesis can be configured to operate according to the routine of FIG. 4. For example, when the prosthesis is operating in the regular mode, it is ready to accept the wake command and ignores all other input commands (voice / touch commands - the prosthesis can be utilized normally). This regular mode can include an active sub-mode and a sleep sub-mode, for example. Indeed, in many scenarios of use, the wake command is entered when in the sleep sub-mode. Then, upon the recognition of a wake command, the prosthesis enters the user interface mode where the device is ready to accept action commands (as distinguished from the wakeup command), and after a certain timeout has elapsed and/or when the aforementioned exit command has been recognized, the prostheses returns to regular mode (which could be the same sub-mode that was left, or to the active sub-mode, for example, as a default, or *vice versa*)*.*

Input from the recipient / user can be tactile and/or voice based. With respect to tactile input, the implant can be configured for touch event recognition. In this regard, touch events can be detected via analysis of energy received into / by the implantable microphone and/or, if available, the accelerometer (some embodiments do not include an accelerometer, and in other embodiments, the accelerometer if present is not used to implement at least some of the teachings herein). Still, while touch event recognition is possible with a single sensor, such as only an implantable microphone, the below assume that implant is equipped with a microphone (i.e. a sensor which can pick up external sound) as well as an "accelerometer" (i.e. a sensor which is mainly sensitive to bone vibrations, but is less sensitive to external sound compared to the microphone sensor). In some embodiments, the microphone and accelerometer are located in a separate housing (more on this below) that is often referred to as an implantable microphone apparatus.

Configurations using an accelerometer, such as the accelerometer detailed above, for example but not by way of limitation, can enable the use of pre-processing techniques, such as those detailed above, to combine a microphone signal and accelerometer signal to improve the event recognition sensitivity (i.e., the true positive rate, or probability of correct detection of actual events) and specificity (i.e., the true negative rate, or probability of correct detection of non-events). If only one of the two sensors can be used, the accelerometer signal can be used over the microphone or *vice versa.* Note that the accelerometer can at least in some instances provide lower false positives relative to utilizing only the microphone (e.g., a hand clap or drum beat will be less likely registered incorrectly as a tap), all other things being equal (note that in some embodiments, any comparison detailed herein is based on the control of all other things being equal). The pre-processed signal(s) is later analyzed with event-recognition algorithms to determine the type of event that has occurred and the confidence/probability of a correct recognition.

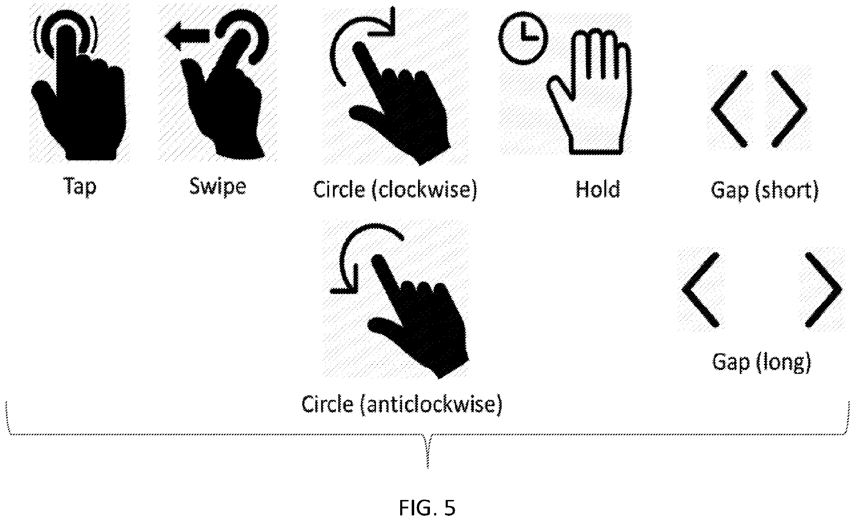

In an exemplary embodiment, the implantable prosthesis can be configured to react to (and thus detect / identify) touch commands that are delivered by the user with motion of a body part (typically, the hand), which do not involve vocalization (in other embodiments, the two can be combined as will be detailed below). These commands can include a sequence and/or combination of events. In this regard, with reference to FIG. 5, the prosthesis can be configured to identify / react to such tactical commands that are based on, for example, tap(s), swipe(s), circle(s) and/or hold(s). Note these are only examples, and embodiments can vary from the disclosure in FIG. 5. FIG. 5 and the teachings thereabout are directed toward conveying the concepts underlying some embodiments / scenarios of use. Differences in input can be utilized. The key is that the device can be, in at least some embodiments, configured to react to the genus and / or species of the concepts disclosed in FIG. 5.

Below are some examples of specific inputs, by way of example and not by way of limitation.

Tap(s) can be short-duration burst(s) that can be generated by tapping on the head, tapping over the skin on top of the implantable microphone, and/or by biting. The number and/or timing of taps can be used to distinguish different commands. By way of example, the sequence [2 taps - gap - 1 tap] can be the "volume up" command while the sequence [3 taps - gap - 1 tap] can be "change program." Note we detail some exemplary embodiments of a gap below.

Swipe(s) can be longer-duration movements that can be generated by sliding/swiping a finger over the head. In some embodiments, the recipient uses his or her finger to draw a line in one or more directions. The direction of the movement can be used to distinguish different commands, e.g. swiping with an upward motion can be the "raise the volume" command, while swiping downward can be the "decrease the volume" command.

Circle(s) can be continuous movement(s) generated by sliding the finger in a circular motion around the skin on top of the implanted microphone. The direction can be used to distinguish different commands, e.g. clockwise motion can be the "raise the volume" command, while anti-clockwise motion can be the "decrease the volume" command.

Hold(s) can correspond to covering the implantable microphone for a certain amount of time (e.g. 3 seconds) using finger(s) or the hand.

The above events can be used alone and/or in combination with each other and/or with voice commands to create sequences of events to assign to a command. Note also that corollaries to voice commands can be used as well. For example, a whistle that slides from low to high and then back to low in a continuous manner might be analogous to a circle or a forwardswipe and then a backswipe.

Additionally, gap(s) can be used, which can correspond to a special event that can be identified by absence of movement (for the microphone, this corresponds to silence) preceding and/or following a tap, swipe, circle, or hold event. In some embodiments, contrary to other events, the "gap" is only used in combination with other events to generate event sequences to assign to a command. The duration of the gap can be used to distinguish between different commands. For example, the sequence [2 taps - short gap - 1 tap] can be the "raise volume" command while the sequence [2 taps - long gap - 1 tap] can be "change program."

In an exemplary embodiment, the prosthesis can be configured to receive and/or react or otherwise detect one or more or all of the above noted types of inputs and/or features of inputs, detailed above, and differentiate between one or more or all of the above noted types of inputs and features of inputs, and to extrapolate a command based on the differentiation. In exemplary embodiments, the prosthesis can be configured to analyze the input, once detected and in some embodiments, determine that such is actual input versus false input, and evaluate such to extrapolate a command or the like based on such input.

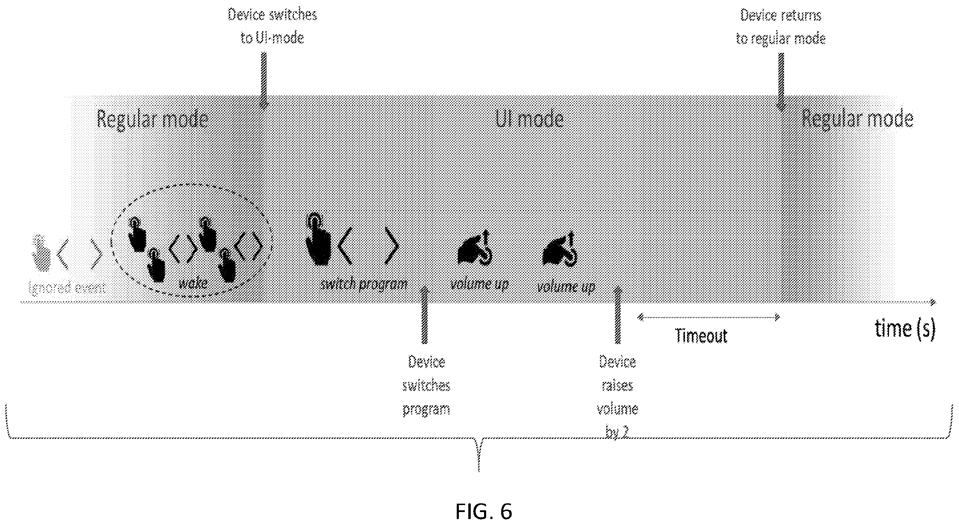

FIG. 6 provides a figure that pictorially represents an exemplary scenario of an exemplary embodiment. In an exemplary embodiment, the prosthesis is configured to receive and analyze and evaluate one or more or all of the inputs and the phenomenon associated therewith (e.g. time between inputs) and extrapolate a desired command therefrom. This is an exemplary embodiment only and presented for illustrations as to the configurations that can be utilized in some embodiments.

In this regard, for purposes of illustrating the functional capabilities by example of an exemplary prosthesis, in an exemplary embodiment, the prosthesis includes 3 programs (P1, P2, P3) in the prosthesis, all at volume 7. (There can be embodiments that include 1, 2, 3, 4, 5, 6, 7, 8, 9, 10, 11, 12, 13, 14, 15 or more programs, or any range of values therebetween in one increment (4-8 programs, for example). In an exemplary scenario of use, the user is listening to P1 but wants to switch to P2 and raise the volume to 9 using the touch user interface. The wake command is [tap - tap - short gap - tap - tap - short gap], the switch-program command is [tap - long gap], and the volume-up command is [swipe-up]. The user interface timeout period is 3 seconds.

At the beginning, the prosthesis is in the regular mode. By accident, the user taps on his or her head once, but no action is taken because this is not the wake command. Then, at some later time, the user enters the wake command. The device recognizes the wakeup command and enters the user interface mode, in which when in the user interface mode, the device accepts action commands. The user enters the switch-program command. The device switches from P1 to P2. Later, the user inputs two volume up commands, and the device raises the volume from 7 to 9. Because the user is happy with these new settings, he/she stops generating touch-events. Because no new event is recognized, after 3 seconds the device returns to regular mode. The user is now on P2 with volume 9.

As noted above, the prosthesis can be configured to receive and react to voice-controlled commands that involve vocalization by the user (similarly to voice commands used with Apple Siri or the Google Assistant). For example, the wake-command could be, for example, the phrase "Hey Cochlear Implant" and actions commands could be "Volume up", "Mute", "Program 1", etc. These commands can be detected by analyzing the implanted microphone signal with a conventional speech recognition algorithm, or similar approaches.

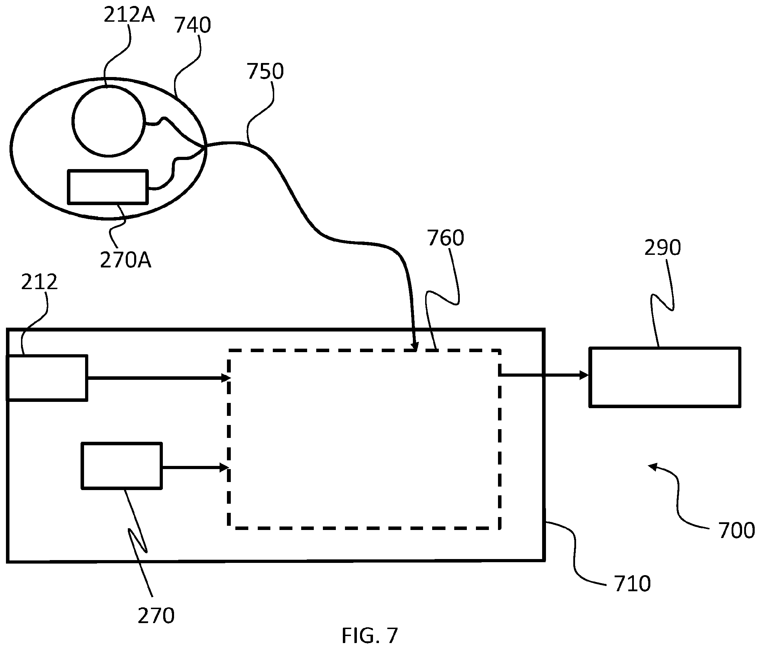

FIG. 7 presents an exemplary totally implantable hearing prosthesis 700 according to an exemplary embodiment. Prosthesis 700 includes a housing 710 which supports or otherwise includes a microphone 212 and/or the accelerometer or other type of sensor 270. Microphone 212 and sensor 270 are in signal communication with processing suite 760, which can include noise cancellation or the like as detailed above, and can include a sound processor, such as a sound processor of a totally implantable hearing prosthesis such as a cochlear implant or an active transcutaneous bone conduction device. Some additional details of the processing suite 760 will be described below.

As seen in FIG. 7, the implantable hearing prosthesis 700 further includes a remote housing 740 that includes or otherwise supports a microphone 212A and/or an accelerometer 270A, one or both of which is in signal communication with the processing suite 760 via cable assembly 750. In an exemplary embodiment, the components of the remote housing 740 are in lieu of the microphone 212 and/or the accelerometer 740. Conversely, in an exemplary embodiment, there is no remote housing and the associated components. Put another way, embodiments can include all of the features seen in FIG. 7 or some of them.

In this exemplary embodiment, the output device, element 290, which can be a cochlear implant electrode array for example, or can be a middle ear actuator or can be a vibrator of an active transcutaneous bone conduction device, by way of example, is shown as being remote from the housing 710 but in signal communication with the processing suite 760. In an exemplary embodiment, element 290 can be located in or with the housing 710.

Returning back to the processing suite. As noted above, the processing suite can include one or more processors or computer chips or memory components or logic circuits or any other type of electronics that can enable the teachings detailed herein. In an exemplary embodiment, there is a cochlear implant sound processor or any of the other processors detailed above that is part of suite 760. In an exemplary embodiment, Suite 760 includes logic circuits or processes or chips that can enable the teachings detailed herein vis-à-vis the wake command or the other commands for example.

In an exemplary embodiment, to input the tactile commands detailed herein into the implanted prosthesis, the user/recipient places his or her fingertip over the skin / touches the skin overlying the microphone and/or sensor at issue / that will be utilized to receive the input. Placing it over / touching is a catch all for the swiping and tapping etc. detailed above. In an exemplary embodiment, this can entail placing the recipient's finger over the housing 710 or over the housing 740. The input can be any input utilizing the recipient's finger(s) that can be received by the various microphones/sensors of the prostheses that can enable command input. Note also that instead of a finger, it could be a fist or could be a palm of a hand that is placed over the microphone or sensor. Any tactile input that can enable the teachings detailed herein can be utilized in some embodiments, and thus in at least some embodiments, the prosthesis can be configured to receive input originating from such tactile input sources.

In an exemplary embodiment, a command can be a command that results from both the microphones and/or sensors of the remote housing 740 and the microphones and/or sensors of the housing 710 receiving the input within a certain temporal proximity of one another. This embodiment can reduce the so-called false positives. By way of example only and not by way of limitation, a wake command could be inputted into the remote housing 740 and then after a period of between two and five seconds a wake command can be inputted into the housing 710, or *vice versa.* If the wake command occurred in both simultaneously, that could be in indicia that it is not a wake command for example, and thus the prosthesis could be configured to ignore such. That is, by way of example, the input that otherwise would be considered a wake command was likely accidental input or another type of input and thus should be disregarded as a wake command because the likelihood of two separate wake commands being received at two separate sensing locations within a specific time period but not simultaneously is relatively low.

Thus, we see that in an exemplary embodiment, the implanted prosthesis can include a housing as noted above with respect to FIG. 1 or FIG. 7 in which the housing includes electronic components configured to enable the prosthesis to operate according to the teachings detailed herein and/or variations thereof. In an exemplary embodiment, the housing is a titanium housing and/or a ceramic housing or any other type of housing that will enable hermetic sealing of the electronic components therein. The housing can include a processor or chip components or other electronic components that can enable the functionality of the prosthesis, such as, for example, hearing prosthesis functionality. In an exemplary embodiment, as noted above, the prosthesis can be a totally implantable hearing prosthesis which can include an implanted/implantable microphone, which microphone can be mounted on the housing or mounted in a separate housing and in signal communication with the housing containing the aforementioned electronics via cables or electrical leads, etc. Still further, in an exemplary embodiment, the prosthesis can be a partially implantable hearing prosthesis which relies on an external component for sound capture and/or to provide power to the implanted component (in the case where there is no implantable power source).

In view of the above, in an exemplary embodiment, there is a device comprising a housing and electronics wherein the device is a prosthesis configured to receive input from a human during a first input reception state and configured to receive input from a human during a second input reception state and operate in a manner not operable during the first input reception state. In an exemplary embodiment, the prosthesis is a sensory prosthesis, such as a hearing prosthesis and/or a retinal prosthesis. Further, in an exemplary embodiment, the first input reception state is a normal input reception state, which can correspond to a reception state that exists during the regular mode of operation noted above. Also in an exemplary embodiment, the second input reception state can be a user interface input state, which can correspond to the user interface mode noted above. Consistent with the teachings above, the prosthesis can be configured to enter the second input state from the first input state upon a human inputting a predetermined command in the first input state, which input is received via a sensor (one or more microphones and/or one or more accelerometers, etc.) of the prosthesis. Also, in an exemplary embodiment, irrespective of the aforementioned sensor, the prosthesis can be configured to respond to one or more predetermined commands in the second input state and configured to not respond to those commands in the first input state. Indeed, in an exemplary embodiment, when in the first input reception state, the only tactile and/or voice input command that will be acknowledged or otherwise reacted to by the prosthesis can be the wake command. No other command will be responded to or otherwise reacted to.

It is briefly noted that in some embodiments, the action of operating in a manner not operable during the first input reception state can be a manner of operation that has utilitarian value with respect to parents or caregivers or the like as opposed to the recipient per se. In an exemplary embodiment, the second state can be a state that is utilized for parent or caregiver purposes. In some exemplary embodiments, the input that constitutes a wake command to transition from the first input state to the second input state is a complicated input that would only be capable of being inputted by an adult or caregiver relative to the recipient, or at least is unlikely to be able to be generated by the recipient because of age or some form of disability. In such exemplary embodiments, this can cause the device to stop functioning (to address a potential sensory overload of a child or the like) or enter a simplified state of operation, etc. In an exemplary embodiment, the various commands detailed below can be inputted by the parent or caregiver or by the recipient for that matter, but the initiation into the user interface state / second input state would likely only be able to be initiated by the parent or caregiver. Such can have utilitarian value with respect to teaching a child on how to use this technology so that the child only initially uses it under the supervision of parents or guardians. In an exemplary embodiment, the complexity of the wake command can be varied or customized so that a less complex input can be utilized in the future allowing the child or the recipient to input the wake command once he or she has become more familiar with utilizing the technology.

Some exemplary embodiments can enable anyone to change or otherwise customize the wake command or any of the commands for that matter. In this regard, in a scenario where the initial wake command causes too many accidental entries into the user interface mode or is such that the recipient has difficulty entering the correct input to enter the user interface mode, the wake command can be changed to another type of command that better suits the recipient and/or the environment.

Accordingly, in an exemplary embodiment, the prosthesis is configured to enter a training or a programming mode that enables the various commands to be customized or set by the recipient or a caregiver so that those specific commands will be utilized after the programming session. This can be done repeatedly or periodically or only one time depending on a given embodiment. In an exemplary embodiment, an external component is configured to provide a signal to the implanted component to indicate that the implanted component is in a programming or a setting or a training mode and thus arrange the implanted component to receive input to "program" the implanted device to recognize certain commands. Indeed, in an exemplary embodiment, the external component can be in communication with a computing device such as a computer or a smart phone or the like. This computing device and/or the external component can include a program or routine that presents different functionalities that can be changed and/or the different operational features of the prosthesis that will be executed upon receipt of the various commands so that the recipient understands what commands he or she wants to correlate there with. This can be an interactive routine or any other routine that can enable the programming of the implanted device or any other pertinent device.

While the embodiments are directed towards typically a single sequence of events that correspond to a wake-up command, there can be a plurality of different types of events that can correspond to the wake-up command.

Note also, in some embodiments, there can be a third input reception state where no tactile and/or voice commands will be responded to, even the wake command. By way of example only and not by way of limitation, in an exemplary embodiment, the tactile commands can be disabled or otherwise the prosthesis can be configured to not respond to any tactile commands when in this third input reception state. To be clear, the concept of a third input reception state corresponds to a reception state where input will not be responded to or otherwise even analyzed. In an exemplary embodiment, this can be utilitarian with respect to embodiments where a recipient wants total control over his or her prosthesis. In an exemplary embodiment, to exit this third input reception state, an external component or the like might be required to be placed into signal communication with the implantable component so as to activate internal logic or circuitry in the implantable portion to enter the first state from the third state, and/or to leave the third state to enter the first state. That said, in an exemplary embodiment, there can be a tactile and/or voice-based command that can be inputted to enter the third state, but a tactile and/or voice-based command cannot be utilized to exit the third state. In an exemplary embodiment, a coin or the like or other metallic component or a magnet for that matter might be utilized to exit the third state or otherwise enter the first state. That said, in an exemplary embodiment, the command to exit the third state and enter the first state could be a tactile command and/or a voice command, but a command that has a very lengthy and/or complicated sequence so as to make it virtually impossible to enter the first state from the third state by accident. By way of example, such could be analogous to a BitLocker code on a computer at boot up, as compared to a simple password. Accordingly, the third input state can be a state requiring a more complicated input, including a substantially more complicated input, relative to that which is the case to enter the second input state from the first input state.

Further, the prosthesis can be configured to respond to one or more predetermined commands in the second input state and configured to not respond to any tactile and/or voice command other than a command that places the prosthesis in the user interface input state when in the first input state and/or other than an emergency command. In some embodiments, the prosthesis is configured to receive input based on tactile input commands when in the first input state (and, in some embodiments, voice, but in others, not voice, while in others, only voice). Also, in some embodiments, the prosthesis is configured to analyze the received input and determine, based on the analysis, whether the input is indicative of a command by a human to enter the second input state and enter the second input state upon a determination that the input is indicative of such a command.

In some embodiments, the prosthesis is a totally implantable prosthesis and the prosthesis is configured to receive energy from tactile input commands that are originated by a human touching skin proximate an implanted sensor of the prosthesis, which energy is used as input in accordance with the teachings herein. In this regard, the tactile input commands can be signals and thus are species of the genus of energy that can be received. As will be detailed herein the prosthesis can be configured to differentiate between body noise energy and command / signal energy.

In some embodiments, the prosthesis is configured to respond to the following in the second input state, and configured to not respond to the following in the first input state: at least 1, 2, 3, 4, 5, 6, 7, 8, 9, 10 or more, or any value or range of values in 1 increment therebetween, different control function inputs and at least 1, 2, 3, 4, 5, 6, 7, 8, 9, 10 or more, or any value or range of values in 1 increment therebetween, status information retrieval request inputs. Further, in some embodiments, the prosthesis is configured to respond to the following command in at least the second input state: exit second input state input. Also, in some embodiments, the prosthesis is configured to automatically exit the second input state after a sequence of inaction within no more than a 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 70, 80, 90, 100, 110, or 120 second period, or any period having values or range of values between any of these in one second increments (e.g., after 105, 106, 112 second, etc.).

In some embodiments, the prosthesis is configured to enter the second input state only upon input during the first input state of a complex coded input, which complex coded input can be definitively more complex, including substantially more complex than the potential complex codes for the other commands that would be reacted to when in the second input state. Still, in an exemplary embodiment, the only complex code is the wake-up code for the wake-up command. In some embodiments, the prosthesis is configured to enter the second input state only upon input during the first input state of a lengthy coded input. That is, the input may not be complex, but is required to be of sufficient length that would differentiate such from other types of input. Thus, an exemplary embodiment, the lengthy coded input can be definitively more lengthy, including substantially more lengthy, than the potential lengthy codes for the other commands that would be reacted to when in the second state. Still, in an exemplary embodiment, the only lengthy coded input could be the code for the command. A combination of the two can be utilized in some embodiments (complex and length).

In at least some exemplary embodiments, the wake-up command can be any command that reduces the likelihood of false positives relative to other commands and/or is a command that is less likely to be mistakenly entered by a recipient relative to other commands that could be entered when in the second state.

In view of the above, it can be seen that in an exemplary embodiment, there is a device, comprising a housing and electronics, wherein the device is a prosthesis configured to receive input from a human via a sensor of the prosthesis, which input is based on tactile input, and evaluate the input to identify a command from a recipient of the prosthesis. This prosthesis can be a hearing prosthesis and/or can be a vision prosthesis, and can be configured to operate in a user interface mode and a normal operation mode, consistent with the teachings detailed above.

In some embodiments, the prosthesis is a hearing prosthesis and/or a vision prosthesis configured to transition from a regular mode to a user interface mode upon tactile and/or voice input and automatically transition back to the regular mode. In an exemplary embodiment, the automatic transition back to regular mode can be after a time elapsed. In an activity, as will be detailed below and/or can be based on some form of logic regarding other types of input, such as a sound input associated with an ambient sound statistically indicative of an environment where the recipient would want to have the prosthesis only operating in the regular mode or otherwise would want the prostheses to be out of the user interface mode. As will be detailed below, in an exemplary embodiment, the prosthesis can be trained or otherwise programmed to operate differently depending on a recipient's desires (timeout can be changed and/or certain sound environments or vision environments can trigger the automatic transition in some instances for some recipients and not others, etc.).

In an exemplary embodiment, there can be an app for smartphones/tablets or other computers that are used for one or more purposes:
1) User training: To teach the user how to use the user interface. This can include a demo movie of each command and a playground-mode for testing commands that gives real-time feedback to the user. For example, the feedback can be a [green light *+ "well done"* message] or [red light + "*try tapping slower"* message].
2) Customize commands: Here the user can choose his/her own sequence of events and assign it to his/her preferred command.
3) Calibration/training of the user interface system. This can be used to improve the accuracy of the event detection/recognition module.
   a. If a touch-user interface is used, touch events can be analyzed by the app to extract useful information that can be sent to the device to train the classification algorithm.
   b. If a voice-controlled user interface is used, the app can be used to build a voice-fingerprint of the user and/or of each voice-command.

Embodiments thus include smart phones or smart tablets or computers or the like configured to enable one or more of the above-noted training and/or customizations.

In an exemplary embodiment, the tactile input upon which the input is based and which the prosthesis is configured to react to includes at least 1, 2, 3 or all of or any value or range of values therebetween in 1 increment of tap input, swipe input, circle input or hold input. Also, in some embodiments, the prosthesis is configured so that it will respond differently to a first of the at least 2, 3 or 4 of the tap input, etc., relative to a second of the at least 2, 3, or 4 of the tap input, etc., and *vice versa.* By way of example only and not by way of limitation, a tap input can result in increased volume and a hold input can result in decreased volume, or *vice versa.*

In an exemplary embodiment of this device, the prosthesis is configured with at least one of electronics sub-components or firmware/software that reduces a false positive evaluation of the input relative to that which would be the case in the absence of the prosthesis being so configured.

In this regard, in an exemplary embodiment, the prosthesis includes one or more of various pre-processing schemes to improve detection rates by emphasizing the signal of interest (taps, swipes, etc.). The approach can be, for example, the attenuation and/or removal of undesired components in the input signal(s). For example, external sounds and own voice would be undesired, and bone vibrations could be desirable.

In an exemplary embodiment, fixed filtering can be used, where a fixed filter is present in / part of the prosthesis that emphasizes one or more frequency bands that are more utilitarian to separate undesired signals from desired ones. A high-pass (or band-pass) filter can be utilized in embodiments where tap and/or swipe signals have more high frequency content than voice, including own voice, which in some embodiments can be considered the main undesired signal. This regime can be used in combination with the other processing schemes. In an exemplary embodiment, the prosthesis can be configured to only evaluate input at certain frequencies, with the idea being that input at other frequencies will not or are unlikely to correspond to a command input. In an exemplary embodiment, this arrangement can be utilized to improve the discrimination between touch events and own voice relative to that which would otherwise be the case in the absence of such filtering. In an exemplary embodiment, if used by itself, the output signal is the accelerometer signal level.

In another exemplary embodiment, accelerometer-to-microphone ratio or cross-correlation monitoring can be used, which can entail monitoring the accelerometer-to-microphone signal power ratio or cross-correlation between accelerometer and microphone signal in order to improve detection specificity. The accelerometer-to-microphone ratio and cross-correlation during touch events will be higher compared to external sounds in some embodiments. By relying on this fact, and evaluating the ratios and/or the correlations, depending on the ratio and/or correlation determined by the prosthesis, an input can be deemed to be a command or at least more likely to be a command. In an exemplary embodiment, this arrangement can be utilized to improve the discrimination between touch events and external sounds, relative to that which would otherwise be the case in the absence of such an arrangement. In some embodiments, the output signal is the accelerometer-to-microphone ratio or accelerometer to microphone cross correlation.

Still further, in an exemplary embodiment, the prosthesis is configured to utilize adaptive filtering algorithms that aid the suppression of residual external sounds from the accelerometer signal. By way of example only and not by way of limitation, least-mean-squares-type algorithms such as the normalized least mean squares (NLMS) can be utilized. In an exemplary embodiment, the adaptive filter can be updated or otherwise is updated only in the presence of external sound. Accordingly, in an exemplary embodiment, the prosthesis is configured to identify the presence of external sound by, by way of example, monitoring the accelerometer-to-microphone ratio. In this exemplary embodiment, this arrangement can be utilized to improve the discrimination between touch events and external sounds relative to that which would otherwise be the case in the absence of such, where here, the output signal is the filtered accelerometer signal.

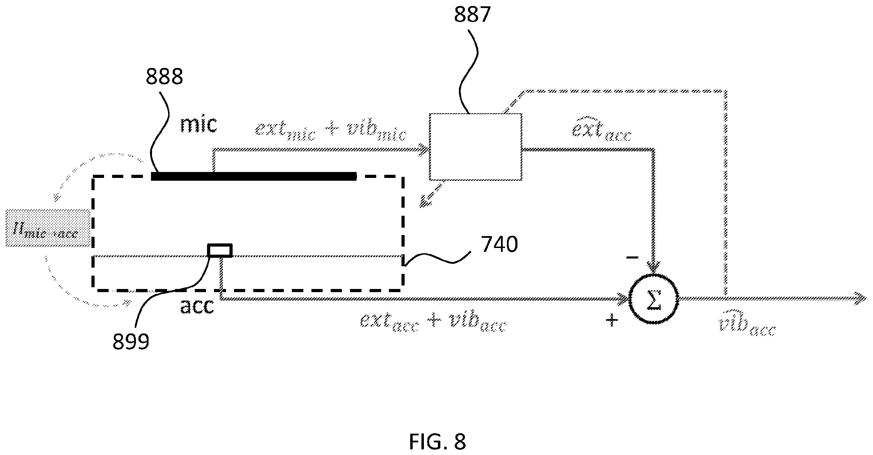

As can be seen, embodiments can utilize pre-existing noise cancellation or adaptive filtering. In this regard, in an exemplary embodiment, the above-noted noise cancellation components can be adapted for use with the teachings herein regarding command identification and/or determination so as to reduce the likelihood of a false positive command input. FIG. 8 depicts a quasi-function diagram of a remote implanted microphone (mic=microphone, acc=accelerometer, element 888 is the microphone diaphragm and element 899 is the accelerometer and element 886 is an adaptive filter/filters) and the adaptive filter 887 used to enhance the signal of interest *vib_{acc}* (body conducted vibrations). In this regard, FIG. 8 represents an exemplary arrangement of an external sound suppression arrangement via adaptive filtering (element 887 are the adaptive filter(s)). It is noted that while the embodiment depicted in FIG. 8 is in reference to the remote microphone, this arrangement can be also applicable to the microphone and the accelerometer that is located in the main housing of the implant. In an exemplary embodiment, the filtered accelerometer signal could be utilized to evaluate the likelihood that the signal is a command input as opposed to another type of noise so as to reduce the likelihood of false positives.

Thus, in an exemplary embodiment, the prosthesis is a totally implantable hearing prosthesis (but the teachings herein are also applicable to a partially implantable hearing prosthesis that has an implantable microphone and/or implantable accelerometer, and can also be applicable to devices where such is not implanted, if the pertinent components are located outside the recipient for example). Here, the sensor is an implantable microphone and the prosthesis also includes an implantable accelerometer. The prosthesis can be configured to evaluate a first signal from the implantable microphone and at least one of evaluate a second signal from the accelerometer or consider the lack of the second signal from the accelerometer to identify the input as a command. Further, in view of the above, the prosthesis can be a hearing prosthesis equipped with a noise cancellation system and the prosthesis can be configured to use components of the noise cancellation system in a manner substantially different from that which is the case for noise cancellation to evaluate the input and increase a probability of a correct detection of the input relative to that which would be the case without such configuration.

By using some or all of the teachings herein, a hearing prosthesis can be configured to discriminate between touch events and own voice events in a statistically significant manner and/or accurate and/or reliable manner (reliable being a statistically analyzed feature) and discriminate between touch events and external sound events in a statistically significant manner. By using some or all of the teachings herein, a hearing prosthesis can be configured to discriminate between touch events and own voice events in an effective / efficacious manner and discriminate between touch events and external sound events in a statistically significant manner.

In view of the teachings herein, embodiments of the prosthesis can be configured with a second reception state safeguard regime that reduces the likelihood of entering the second reception state accidentally by at least 10, 15, 20, 25, 30, 35, 40, 45, 50, 55, 60, 65, 70, 75, 80, 85, 90, 95 or 100% or any value or range of values therebetween in 1 % increments, relative to that which would otherwise be the case in the absence of this safeguard regime.

In view of the teachings herein, embodiments of the prosthesis can be configured with a second reception state safeguard regime that reduces occurrences of entering the second reception state accidentally by at least 10, 15, 20, 25, 30, 35, 40, 45, 50, 55, 60, 65, 70, 75, 80, 85, 90, 95, or 100% or any value or range of values therebetween in 1% increments, relative to that which would otherwise be the case in the absence of this safeguard regime, over a period of one (1) month of consistent use after one (1) year of consistent use (to account for acclimation).

Embodiments can use a sensitivity parameter controllable by the user and/or the user's caregiver and/or health-care professional following / studying / working with the user. This parameter is used to revise the detection and/or classification algorithm and/or its parameters in order to improve accuracy in some embodiments. Accordingly, in an exemplary embodiment, the prosthesis is configured to enable input of the sensitivity level / adjust the sensitivity to the input.

Thus, in view of the above, embodiments include command event recognition regimes / prostheses configured to execute a recognition regime to detect / recognize input commands. In this regard, an embodiment includes preprocessing signals according to any of the embodiments herein or embodiments can enable the teachings herein, such as for example, using a filtered accelerometer envelope signal. Embodiments can be prostheses configured to execute detection regimes that:
- detect if a command event has occurred / has not occurred
   o is it a real event or just noise?
- classify the type of the event
   o e.g., is it a tap or a swipe?
- optionally, estimate the probability/likelihood of a correct detection
   o can be used to adjust the user interface sensitivity
   o this information can be logged for later analysis to revise the detection and/or classification algorithms in order to improve accuracy

Note that these three steps need not necessarily to be separate.

An exemplary embodiment includes utilizing algorithms to implement the above, such as, for example, using matched filtering, decision trees, nearest neighbors, logistic regression, Markov models, Bayesian classifiers and/or neural networks.

In some embodiments, the prosthesis is configured to evaluate a signal duration and/or amplitude (including its time-modulations and frequency-modulations) and use such evaluation to directly perform the detection/recognition and/or to train an algorithm to do so.

If no training data are available, some embodiments can utilize event detection via estimation of the "noise" level (where the noise is represented by the signals that are not of interest), computation of the signal-to-noise ratio, and the utilization of simple thresholding to decide if there was an event or not an event. An alternative approach is applying thresholding directly on the (preprocessed) signal (thus skipping the noise and signal-to-noise ratio estimation). Also, event classification can be used, such as once the signal of interest is isolated from the noise, the signal duration is extracted, and the amplitude and its changes in time/frequency are identified and a simple decision tree can be used to determine the event type.

In an exemplary embodiment, there is a touch-events based user interface capable of recognizing taps and/or swipes. Here, an input signal can be an unprocessed accelerometer signal outputted as a result of a tap or a swipe on the skin in proximity to the housing containing the accelerometer. The prosthesis can be configured to implement preprocessing, such as the utilization of high-pass filtering. The preprocessing can also include the estimation of the accelerometer envelope signal at relatively slow rates (e.g., 100-250 Hz).

The prosthesis can also be configured to detect events. The prosthesis can be configured to estimate the "noise" level in the (processed / filtered) accelerometer as the median of the accelerometer envelope signal for the computation of signal-to-median ratio (SMR), and using the thresholding to distinguish between events and non-events, such as setting a threshold of 20 dB.

The prosthesis can also be configured to implement event classification, where, for example, the event duration can be utilized to classify taps and swipes. For example, swipes will be classified as such under an event of duration = 50 - 500 ms, and a tap will be classified as such if the duration is 10-40 ms. In some embodiments, for example, if two (2) taps are recognized within 20 ms, only the second tap is registered. Also, the average and/or maximum SMR can be utilized to derive an estimate of the probability of a correct recognition.

Thus, in view of the above, there is a touch controlled user interface that is event based for a prosthesis. In some embodiments there are predetermined fixed sequences of events corresponding to taps, swipes, and gaps. In an exemplary embodiment, the prosthesis can be configured so that the wake command = [tap - tap - short gap - tap - tap - long gap], the volume up command = [tap - tap - tap - long gap], the volume down command = [tap - tap - long gap], the switch to next program command = [swipe - long gap], the retrieve battery status command = [swipe - swipe - long gap]. In some embodiments, swipes are direction independent and long gaps are used to indicate that user input of a command is finished.

An exemplary algorithm used by the prosthesis that can enable the teachings herein can rely on processing where fixed plus adaptive filtering is used, including for example pre-processing, event detection utilizing, for example, thresholding on the signal-to-noise ratio (SNR) estimate to determine / differentiate between an accidental / non command and an intentional command. Note that the "noise" is the undesired signal (own voice and external sound) rather than "ambient noise." Embodiments utilize event classification, which can use a simple decision tree to determine the event type. Input features to the tree can be, for example, signal duration and its amplitude changes in time (e.g., amplitude at the beginning of the duration-window, middle, and end, max duration, etc.), all used to determine / identify the event (swipe, tap, etc.). The prosthesis can be configured to determine the likelihood of correct classification for each event and/or utilize thresholding on the probability of correct detection to determine such. For each recognition, in an exemplary embodiment, an estimate of the probability of correct detection (i.e., how confident that what was recognized as a tap, for example, is truly a tap) can be developed by the prosthesis, using logic circuitry, including artificial intelligence or learning, or training programs, etc. Some embodiments can utilize the thresholding on this probability to ignore weak recognitions (and thus lower false positive recognition rate).

And, as noted above, a computer based program, such as a smartphone app can be used to train the user to use the touch user interface and to calibrate the sensitivity of the event detection/recognition algorithms.

Some embodiments can include a prosthesis configured to receive and identify a sequence of input to alert the device to incoming commands, and also a sequence command (like voice command on iPhone). A pre-sequence to prepare the device for an incoming info signal can be implemented. This can prevent a false positive.

Some embodiments can include an "exit sequence" to get out of the second state, which can be a voice command such as "I don't want to send any more commands" or any other command that can be utilitarian.

Accordingly, in view of the above, the aforementioned device can be such that the prosthesis is configured with at least one of electronics sub-components or firmware/software that reduces a false positive evaluation of the input relative to that which would be the case in the absence of the prosthesis being so configured. In an exemplary embodiment, for ten different inputs that result in false positives, the reduction of false positives can be 10, 15, 20, 25, 30, 35, 40, 45, 50, 55, 60, 65, 70, 75, 80, 85, 90, 95 or 100% or any value or range of values therebetween in 1 % increments, relative to that which would otherwise be the case, if those inputs are repeated using the false positive reduction techniques detailed herein.

While embodiments have been directed towards the utilization of a totally implantable hearing prosthesis as the device that receives the input, or embodiments of a partially implantable hearing prostheses that includes an implanted sensor or sensors that receives the input, some embodiments can include an external device that includes external sensors not implanted in the recipient which receive the input. In an exemplary embodiment, a BTE device or the like can be the device that receives the input in the form of a tap or swipe or other tactile or voice-based input. This embodiment can have utilitarian value with respect to freeing the recipient from having to utilize buttons or other input to obtain data or otherwise control the function of the prosthesis or otherwise change a function or change a setting of the prosthesis. To be clear, while the embodiments of the teachings detailed herein appear to have greatest utility with respect to devices that have implanted sensors, such as totally implantable hearing prostheses, it is possible that there is utilitarian value with respect to utilizing these teachings and an external device. Accordingly, embodiments include an external device that includes external sensors, such as a BTE device, with a tactile input and/or voice inputs that are inputted into that external device.

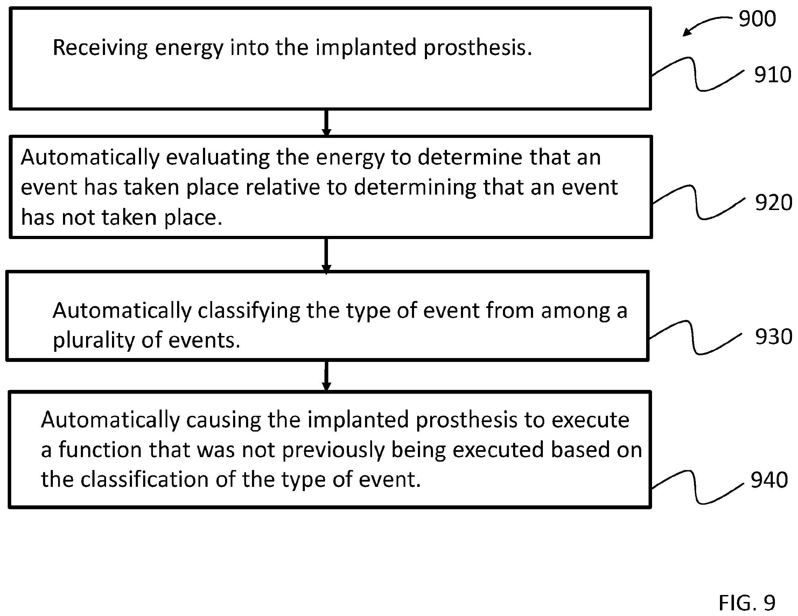

FIG. 9 presents an exemplary flowchart 900 for an exemplary algorithm for an exemplary method that can be implemented in accordance with the teachings herein. In an exemplary embodiment, this method is implemented entirely in an implanted prosthesis, while in other embodiments can implement this in a prosthesis that is not entirely implanted. Method 900 includes method action 910, which includes receiving energy into the implanted prosthesis (or into another part of the prosthesis in embodiments that are not implanted). This can correspond to the above-noted taps or swipes or voice input, etc. This can also correspond to background noise or the normal operation of the prostheses. To be clear, in an exemplary embodiment, the energy that is received and the prosthesis is received via an implanted microphone and/or an implanted accelerometer or some other implanted sensor that typically receives energy during normal use of the prosthesis.

Method 900 further includes method action 920, which includes the action of automatically evaluating the energy to determine that an event has taken place relative to determining that an event has not taken place. As detailed above, the prosthesis will receive energy including unwanted energy due to body conducted noise and due to ambient environments, etc. utilizing the teachings detailed herein, that energy is evaluated to identify the energy as an event pertinent to the identification of a wake command or the like. In this regard, in at least some exemplary embodiments, the action of automatically evaluating the energy is continuous. That said, in some embodiments, the action of automatically evaluating the energy is such that the prosthesis only does so in certain states of the regular mode. By way of example only and not by way of limitation, if the prosthesis logic determines that the prosthesis is being actively used for hearing purposes and it is unlikely that the recipient will want to utilize the user interface at a given time, it may not constantly automatically evaluate received energy. That said, in alternate embodiments, the prosthesis is always actively evaluating the energy to make the aforementioned determination.

Method 900 further includes method action 930, which includes automatically classifying the type of event from among a plurality of events and also includes method action 940, which includes, automatically causing the implanted prosthesis to execute a function that was not previously being executed based on the classification of the type of event.

The classification can be executed utilizing the teachings detailed above and variations thereof. To be clear, the recited classification is different than a sound /auditory scene or sight scene classification or the like, or more accurately, the antecedent action of evaluating the energy to determine that an event has taken place differentiates the classification from sound / auditory scene classification by way of example.

The action of automatically causing the prosthesis to execute a function in accordance with method action 940 can correspond to any of the functions detailed herein and/or variations thereof.

Referring back to method action 910, the action of receiving energy into the implanted prosthesis is executed, with respect to a hearing prosthesis, via energy received by the microphone and/or the accelerometer, as distinguished from, for example, energy received by an inductance coil or energy received via an electrical signal, etc. To be clear, in the embodiment of method 900, the energy received is not an electromagnetic signal. In an exemplary embodiment, the energy received is purely pressure based / vibration based.

Embodiments can include variations of method 900, such as a method that includes the action of automatically estimating a probability and/or likelihood that the detection and or evaluating and/or classification is correct. Such can be executed in accordance with the teachings detailed above and variations thereof. Any device, system, and/or method that can enable the automatic estimation of such can be utilized in some embodiments providing that such has utilitarian value and is enabled by the art. In an exemplary embodiment, method 900 further includes, based on the estimation, determining whether or not to automatically cause the implanted prosthesis to execute the aforementioned function of method action 940. Accordingly, in an exemplary embodiment, there exist a method where the probability and/or the likelihood that is estimated achieves or is higher than a predetermined threshold and thus the method proceeds to method action 940, whereas the method would not have proceeded to such if the predetermined threshold had not been reached.

As noted above, various training techniques can be utilized to improve the classification and/or detection executed by the prosthesis to avoid or otherwise reduce the likelihood of false positives. By way of example only and not by way of limitation, a data logging regime can be included in the prosthesis, where, for example, the prosthesis is configured to data log utilizing onboard memory and/or utilizing wireless transmission to a remote device, such as a handheld smart phone or a user assistant device common with an implanted hearing prosthesis, where the data is logged. In an exemplary embodiment, there is a method of logging data based on the actions of evaluating, classifying, and causing of method 900. In an exemplary embodiment, the prosthesis can be configured to receive input indicative of whether or not the function that is executed in method action 940 was desired by the recipient and/or otherwise can be configured to deduce utilizing latent variables whether such was the case (e.g., if the recipient changes the volume back to what it previously was prior to the implementation of the function of method 940 within a certain timeframe, such can be indicative of a mistake and the logic of the prostheses would log that such was an incorrect or otherwise unwanted executed function, and thus correlate the sequence of events that led to that with such in a data logging procedure). Embodiments can include the action of revising an algorithm that is utilized to execute the automatic estimation of the probability and/or likelihood based on the logging data. In this regard, if the data that is logged indicates that the sequence of events that resulted in the classification of the type of event was not that which should have resulted in the classification of that event, the algorithm will be revised so that if such input is seen again, the classification could be different or no classification at all or in fact that the action of evaluating the energy to determine that an event has taken place would result in a determination that the event has not taken place. Of course, corollary to this is a scenario where if input is not resulting in the action of automatically causing the implanted prosthesis to execute a function when the recipient in fact wants that function to be executed, such can also be logged and these data can be utilized to revise the algorithm. Note that "revising an algorithm" can include replacement of the algorithm by updating the algorithm with a new algorithm or a new portion of the algorithm and/or parameters thereof (e.g., the algorithm can be the same, but one or more parameters can have changed, such as, for example, the energy threshold to distinguish commands form noise), which could be executed by the recipient initiating such during periodic upgrades to the prostheses. Also, in some embodiments, the prosthesis or a hand held assistant or the device that is in communication with the prosthesis, such as a smart phone or the like, could also do the evaluation of the data and develop a revision to the algorithm, where the action by the recipients of permitting or enabling the communication of the prosthesis with this remote device would constitute the action of revising the algorithm if that algorithm is revised based on an update from this remote device.

Still further, a variation of method 900 includes the additional action of executing, by a recipient of the implanted device, a training and/or calibration method that, upon the completion thereof, improves the efficacy of an evaluation algorithm and/or classification algorithm used to execute the evaluation and/or classification, relative to that which would be the case.

An exemplary embodiment of method 900 further include the action of executing a fail-safe procedure (e.g., a safeguard against an undesired implementation of method action 940) based on two separate energy inputs into the implanted prosthesis that originate from different phenomenon in kind or phenomenon entering a body at which the implant is implanted at different locations or phenomenon that occur at temporally disparate locations. Further, an exemplary embodiment of method 900 further include the action of executing a fail-safe procedure based on three separate energy inputs into the implanted prosthesis that originate from different phenomenon in kind (e.g., tap, voice and head tilting) or phenomenon entering a body at which the implant is implanted at different locations or phenomenon that occur at temporally disparate locations. Also, an exemplary embodiment of method 940 includes the action of executing a confirmation action prior to the action of automatically causing the implanted prosthesis to execute a function.

In some exemplary embodiments, the prosthesis can include a processor or other electronics, such as logic circuitry and computer chips, that is / are configured to access software, firmware, and/or hardware that is "programmed" or otherwise configured to execute one or more of the aforementioned functions herein. By way of example only and not by way of limitation, the processor apparatus can include hardware in the form of circuits / circuitry and processing units that are configured to enable the analysis detailed above and/or below, the output of such being utilized to execute the teachings detailed herein. In some embodiments, the processor apparatus utilizes analog circuits and/or digital signal processing such as the fast Fourier transform. The processor apparatus or other device can be configured to execute an algorithm of one of the many various "digital signal processing" techniques available to implement the teachings herein. Note also that existing analog circuits can be used, including those that execute the fast Fourier transform. The prosthesis can be configured to utilize lookup tables or preprogrammed logic or even artificial intelligence systems to implement various method actions / functionalities herein. The programming / code can be located in hardware, firmware and/or software.

Accordingly, embodiments include a prosthesis that includes an electronics package corresponding to modern processors or electronic circuits or the like that is configured to execute one or more or all of the detailed functions herein in an automatic manner. Any electronics assembly that can enable the teachings detailed herein can be utilized in at least some exemplary embodiments. Logic circuits established by silicon chips can be utilized to implement at least some of the teachings detailed herein, which logic circuits can be located in the housing of the prosthesis as detailed above.

It is noted that any method detailed herein also corresponds to a disclosure of a device and/or system configured to execute one or more or all of the method actions associated with the device and/or system as detailed herein. In an exemplary embodiment, this device and/or system is configured to execute one or more or all of the method actions in an automated fashion. That said, in an alternate embodiment, the device and/or system is configured to execute one or more or all of the method actions after being prompted by a human being. It is further noted that any disclosure of a device and/or system detailed herein corresponds to a method of making and/or using that device and/or system, including a method of using that device according to the functionality detailed herein.

Any action disclosed herein that is executed by the implanted prosthesis can be executed by a portion of the prosthesis that is external to the recipient and/or another device, such as a smart watch or a smart phone or a laptop or desktop computer or a remote server and/or another component of any system detailed herein in an alternative embodiment, unless otherwise noted or unless the art does not enable such. By way of example only and not by way of limitation, an embodiment could utilize Bluetooth technology that is part of the implant to communicate with a device that is remote from the implant, such as a smart phone or some other device that is located remote from the prostheses. In an exemplary embodiment, data based on the raw input resulting from method action 910 could be transmitted wirelessly to this remote device, and, for example, the remote device can execute method actions 920 and/or 930, and then communicate back to the implant a command to execute method action 940. This embodiment can be utilized while, for example, sleeping or the like, where the recipient will be relatively stationary and in close proximity to this remote device.

In an exemplary embodiment, a charging device that is utilized to charge the totally implantable device could be utilized to execute one or more of method actions 920 and/or 930, where the raw data based on method action 910 is communicated there to.

Thus, at least some of the functionality of the prosthesis can be present in a smart watch and/or a smart phone and/or another component of any system in an alternative embodiment, all providing that the art enables such.

It is further noted that any disclosure of a device and/or system detailed herein also corresponds to a disclosure of otherwise providing that device and/or system.

It is also noted that any disclosure herein of any process of manufacturing and/or providing a device corresponds to a device and/or system that results therefrom. It is also noted that any disclosure herein of any device and/or system corresponds to a disclosure of a method of producing or otherwise providing or otherwise making such.

Any embodiment or any feature disclosed herein can be combined with any one or more or other embodiments and/or other features disclosed herein, unless explicitly indicated and/or unless the art does not enable such. Any embodiment or any feature disclosed herein can be explicitly excluded from use with any one or more other embodiments and/or other features disclosed herein, unless explicitly indicated that such is combined and/or unless the art does not enable such exclusion.

While various embodiments of the present invention have been described above, it should be understood that they have been presented by way of example only, and not limitation. It will be apparent to persons skilled in the relevant art that various changes in form and detail can be made therein without departing from the scope of the invention.

## Claims

1. A device, comprising:
a housing (710); and
electronics, wherein:
the device is a prosthesis (700) configured to receive input from a human during a first input reception state and configured to receive input from a human during a second input reception state and operate in a manner not operable during the first input reception state;
**characterized in that**
the prosthesis (700) is configured with a second reception state safeguard regime that reduces the likelihood of entering the second input reception state accidentally relative to that which would be the case without the second reception state safeguard regime.

2. The device of claim 1, wherein:
the prosthesis (700) is a sensory prosthesis;
the first input reception state is a normal input reception state;
the second input reception state is a user interface input state; and
the prosthesis (700) is configured to enter the second input reception state from the first input reception state upon a human inputting a predetermined command in the first input reception state; and
the prosthesis (700) is configured to respond to one or more predetermined commands in the second input reception state and configured to not respond to those commands in the first input reception state.

3. The device of claim 2, wherein:
the prosthesis (700) is configured to not respond to any tactile and/or voice command other than a command that places the prosthesis (700) in the user interface input state when in the first input reception state and/or other than an emergency command.

4. The device of claim 2, wherein:
the prosthesis (700) is configured to respond to the following in the second input reception state, and configured to not respond to the following in the first input reception state:
at least three different control function inputs;
at least one status information retrieval request input;
the prosthesis (700) is configured to respond to the following command in at least the second input state: exit second input state input; and
the prosthesis (700) is configured to automatically exit the second input reception state after a sequence of inaction within no more than a 20 second period.

5. The device of any one of claims 1 to 4, wherein:
the prosthesis (700) is configured to enter the second input reception state only upon input during the first input reception state of a complex coded input.

6. The device of any one of claims 1 to 5, wherein:
the prosthesis (700) is configured to receive input based on tactile input commands when in the first input reception state; and
the prosthesis (700) is configured to analyze the received input and determine, based on the analysis, whether the input is indicative of a command by a human to enter the second input reception state and enter the second input reception state upon a determination that the input is indicative of such a command.

7. The device of any one of claims 1 to 6, wherein:
the prosthesis (700) is a totally implantable prosthesis; and
the tactile input commands are originated by a human touching skin proximate an implanted sensor of the prosthesis (700).

8. The device according to any one of claims 1 to 7, wherein the input is based on tactile input, and the prosthesis (700) is configured to evaluate the input to identify a command from a recipient of the prosthesis (700).

9. The device of claim 8, wherein:
the prosthesis (700) is configured with at least one of electronics sub-components or firmware/software that reduces a false positive evaluation of the input relative to that which would be the case in the absence of the prosthesis (700) being so configured.

10. The device of claim 8 or claim 9, wherein:
the tactile input upon which the input is based and which the prosthesis (700) is configured to react to includes at least two of tap input, swipe input, circle input or hold input; and
the prosthesis (700) is configured so that it will respond differently to a first of the at least two of the tap input, swipe input, circle input or hold input, relative to a second of the at least two of the tap input, swipe input, circle input or hold input, and *vice versa.*

11. The device of any one of claims 8 to 10, wherein:
the prosthesis (700) is a totally implantable hearing prosthesis:
the sensor is an implantable microphone (212);
the prosthesis (700) also includes an implantable accelerometer (270);
the prosthesis (700) is configured to evaluate a first signal from the implantable microphone (212) and at least one of evaluate a second signal from the accelerometer (270) or consider the lack of the second signal from the accelerometer (270) to identify the input as a command.

12. The device of any one of claims 8 to 10, wherein:
the prosthesis (700) is a totally implantable hearing prosthesis;
the sensor is an implantable microphone (212);
the prosthesis (700) is devoid of an accelerometer (270).

13. The device of any one of claims 8 to 12, wherein:
the prosthesis (700) is a hearing prosthesis equipped with a noise cancellation system; and
the prosthesis (700) is configured to use components of the noise cancellation system in a manner substantially different from that which is the case for noise cancellation to evaluate the input and increase a probability of a correct detection of the input relative to that which would be the case without such configuration.

14. The device of any one of claims 8 to 13, wherein:
the prosthesis (700) is a hearing prosthesis configured to discriminate between touch events and own voice events in a statistically significant manner and discriminate between touch events and external sound events in a statistically significant manner; and/or
the prosthesis (700) is configured with at least one of electronics sub-components or firmware/software that looks for tactile input comprising an order followed by a reverse order to identify a command from a recipient of the prosthesis (700).

15. The device of any one of claims 1 to 14, wherein the second reception state safeguard regime reduces the likelihood of entering the second input reception state accidentally by at least 50% relative to that which would be the case without the second reception state safeguard regime.

## Patentansprüche

1. Vorrichtung, die umfasst:
ein Gehäuse (710); sowie Elektronik, wobei:
die Vorrichtung eine Prothese (700) ist, die so ausgeführt ist, dass sie während eines ersten Eingabe-Empfangszustandes Eingaben von einem Menschen empfängt, und so ausgeführt ist, dass sie während eines zweiten Eingabe-Empfangszustandes Eingaben von einem Menschen empfängt und so arbeitet, wie sie während des ersten Eingabe-Empfangszustandes nicht arbeiten kann,
**dadurch gekennzeichnet, dass**
die Prothese (700) mit einem Schutzregime für den zweiten Empfangszustand konfiguriert ist, das die Wahrscheinlichkeit von versehentlichem Eintreten in den zweiten Eingabe-Empfangszustand verglichen mit gegenüber denjenigen reduziert, die ohne das Schutzregime für den zweiten Empfangszustand gegeben wäre.

2. Vorrichtung nach Anspruch 1, wobei:
die Prothese (700) eine sensorische Prothese ist;
der erste Eingabe-Empfangszustand ein normaler Eingabe-Empfangszustand ist;
der zweite Eingabe-Empfangszustand ein Benutzerschnittstellen-Eingabezustand ist; und die Prothese (700) so ausgeführt ist, dass sie, wenn in dem ersten Eingabe-Empfangszustand ein Mensch eine vorgegebene Anweisung eingibt, von dem ersten Eingabe-Empfangszustand ausgehend in den zweiten Eingabe-Empfangszustand eintritt; und
die Prothese (700) so ausgeführt ist, dass sie auf eine oder mehrere vorgegebene Anweisung/en in dem zweiten Eingabe-Empfangszustand reagiert, und so ausgeführt ist, dass sie auf die Anweisungen in dem ersten Eingabe-Empfangszustand nicht reagiert.

3. Vorrichtung nach Anspruch 2, wobei:
die Prothese (700) so ausgeführt ist, dass sie auf keine andere Berührungs- und/oder Sprachanweisung als eine Anweisung, die die Prothese (700), wenn sie sich in dem ersten Eingabe-Empfangszustand befindet, in den Benutzerschnittstellen-Eingabezustand versetzt, und/oder als eine Notfallanweisung reagiert.

4. Vorrichtung nach Anspruch 2, wobei:
die Prothese (700) so ausgeführt ist, dass sie in dem zweiten Eingabe-Empfangszustand auf die folgenden Eingaben reagiert, und so ausgeführt ist, dass sie in dem ersten Eingabe-Empfangszustand nicht auf die folgenden Eingaben reagiert:
wenigstens drei unterschiedliche Steuerungsfunktions-Eingaben;
wenigstens eine Eingabe zum Anfordern von Statusinformations-Abruf;
die Prothese (700) so ausgeführt ist, dass sie auf die folgende Anweisung wenigstens in dem zweiten Eingabezustand reagiert:
Eingabe zum Verlassen des zweiten Eingabezustandes; und
die Prothese (700) so ausgeführt ist, dass sie den zweiten Eingabe-Empfangszustand nach einer Sequenz von Inaktivität innerhalb eines Zeitraums von nicht mehr als 20 Sekunden automatisch verlässt.

5. Vorrichtung nach einem der Ansprüche 1 bis 4, wobei:
die Prothese (700) so ausgeführt ist, dass sie nur auf Eingeben einer komplexen codierten Eingabe während des ersten Eingabe-Empfangszustandes hin in den zweiten Eingabe-Empfangszustand eintritt.

6. Vorrichtung nach einem der Ansprüche 1 bis 5, wobei:
die Prothese (700) so ausgeführt ist, dass sie Eingaben auf Basis von Berührungseingabe-Anweisungen empfängt, wenn sie sich in dem ersten Eingabe-Empfangszustand befindet; und
die Prothese (700) so ausgeführt ist, dass sie die empfangene Eingabe analysiert und auf Basis der Analyse feststellt, ob die Eingabe eine Anweisung zum Eintreten in den zweiten Eingabe-Empfangszustand durch einen Menschen anzeigt, und auf Feststellung dahingehend hin, dass die Eingabe eine derartige Anweisung anzeigt, in den zweiten Eingabe-Empfangszustand eintritt.

7. Vorrichtung nach einem der Ansprüche 1 bis 6, wobei
die Prothese (700) eine vollständig implantierbare Prothese ist; und
die Berührungseingabe-Anweisungen von einem Menschen stammen, der Haut nahe an einem implantierten Sensor der Prothese (700) berührt.

8. Vorrichtung nach einem der Ansprüche 1 bis 7, wobei die Eingabe auf Berührungseingabe basiert und die Prothese (700) so ausgeführt ist, dass sie die Eingabe bewertet, um eine Anweisung von einem Träger der Prothese (700) zu identifizieren.

9. Vorrichtung nach Anspruch 8, wobei:
die Prothese (700) wenigstens mit Elektronik-Teilkomponenten oder Firmware/Software ausgeführt ist, die eine falsche positive Bewertung der Eingabe im Vergleich zu derjenigen reduziert, die vorliegen würde, wenn die Prothese (700) nicht so ausgeführt wäre.

10. Vorrichtung nach Anspruch 8 oder Anspruch 9, wobei:
die Berührungseingabe, auf der die Eingabe basiert und auf die die Prothese (700) ausführungsgemäß reagiert, wenigstens zwei Eingaben von Tipp-Eingabe, Wisch-Eingabe, Kreis-Eingabe oder Halte-Eingabe einschließt; und
die Prothese (700) so ausgeführt ist, dass sie auf eine erste der wenigstens zwei Eingaben von der Tipp-Eingabe, Wisch-Eingabe, Kreis-Eingabe oder Halte-Eingabe anders reagiert als auf eine zweite der wenigstens zwei Eingaben von der Tipp-Eingabe, Wisch-Eingabe, Kreis-Eingabe oder Halte-Eingabe und umgekehrt.

11. Vorrichtung nach einem der Ansprüche 8 bis 10, wobei:
die Prothese (700) eine vollständig implantierbare Hörprothese ist;
der Sensor ein implantierbares Mikrofon (212) ist;
die Prothese (700) des Weiteren einen implantierbaren Beschleunigungsmesser (270) einschließt;
die Prothese (700) so ausgeführt ist, dass sie ein erstes Signal von dem implantierbaren Mikrofon (212) bewertet und wenigstens ein zweites Signal von dem Beschleunigungsmesser (270) bewertet oder das Fehlen des zweiten Signals von dem Beschleunigungsmesser (270) zum Identifizieren der Eingabe als eine Anweisung berücksichtigt.

12. Vorrichtung nach einem der Ansprüche 8 bis 10, wobei:
die Prothese (700) eine vollständig implantierbare Hörprothese ist;
der Sensor ein implantierbares Mikrofon (212) ist;
die Prothese (700) keinen Beschleunigungsmesser (270) aufweist.

13. Vorrichtung nach einem der Ansprüche 8 bis 12, wobei:
die Prothese (700) eine Hörprothese ist, die mit einem Geräuschunterdrückungssystem ausgestattet ist; und
die Prothese (700) so ausgeführt ist, dass sie Komponenten des Geräuschunterdrückungssystems auf eine Weise nutzt, die sich im Wesentlichen von derjenigen bei Geräuschunterdrückung unterscheidet, um die Eingabe zu bewerten und eine Wahrscheinlichkeit einer korrekten Erfassung der Eingabe verglichen mit derjenigen in dem Fall zu verbessern, in dem keine derartige Ausführung vorliegt.

14. Vorrichtung nach einem der Ansprüche 8 bis 13, wobei:
die Prothese (700) eine Hörprothese ist, die so ausgeführt ist, dass sie zwischen Berührungsereignissen und eigenen Stimmereignissen in einer statistisch signifikanten Weise unterscheidet und zwischen Berührungsereignissen und externen Schallereignissen in einer statistisch signifikanten Weise unterscheidet; und/oder
die Prothese (700) wenigstens mit Elektronik-Teilkomponenten oder Firmware/Software ausgeführt ist, die nach Berührungseingabe sucht, die eine Reihenfolge, gefolgt von einer umgekehrten Reihenfolge umfasst, um eine Anweisung von einem Träger der Prothese (700) zu identifizieren.

15. Vorrichtung nach einem der Ansprüche 1 bis 14, wobei das Schutzregime für den zweiten Empfangszustand die Wahrscheinlichkeit unbeabsichtigten Eintretens in den zweiten Eingabe-Empfangszustand um wenigstens 50 % verglichen mit derjenigen reduziert, die ohne das Schutzregime für den zweiten Empfangszustand vorläge.

## Revendications

1. Dispositif, comprenant :
un boîtier (710) ; et
de l'électronique, dans lequel :
le dispositif est une prothèse (700) configurée pour recevoir une entrée provenant d'un humain pendant un premier état de réception d'entrée et configurée pour recevoir une entrée provenant d'un humain pendant un second état de réception d'entrée et fonctionner d'une manière non utilisable pendant le premier état de réception d'entrée ;
**caractérisé en ce que**
la prothèse (700) est configurée avec un mécanisme de protection du second état de réception qui réduit la probabilité de passer accidentellement dans le second état de réception d'entrée, par rapport à ce qui serait le cas sans le mécanisme de protection du second état de réception.

2. Procédé selon la revendication 1, dans lequel :
la prothèse (700) est une prothèse sensorielle ;
le premier état de réception d'entrée est un état de réception d'entrée normal ;
le second état de réception d'entrée est un état d'entrée d'interface utilisateur ; et
la prothèse (700) est configurée pour passer dans le second état de réception d'entrée à partir du premier état de réception d'entrée lorsqu'un humain entre une commande prédéterminée dans le premier état de réception d'entrée ; et
la prothèse (700) est configurée pour réagir à une ou plusieurs commandes prédéterminées dans le second état de réception d'entrée et configurée pour ne pas réagir à ces commandes dans le premier état de réception d'entrée.

3. Procédé selon la revendication 2, dans lequel :
la prothèse (700) est configurée pour ne pas réagir à une quelconque commande tactile et/ou vocale autre qu'une commande qui place la prothèse (700) dans l'état d'entrée d'interface utilisateur quand elle se trouve dans le premier état de réception d'entrée et/ou autre qu'une commande d'urgence.

4. Dispositif selon la revendication 2, dans lequel :
la prothèse (700) est configurée pour réagir à ce qui suit dans le second état de réception d'entrée, et configurée pour ne pas réagir à ce qui suit dans le premier état de réception d'entrée :
au moins trois entrées de fonction de commande différentes ;
au moins une entrée de demande d'obtention d'informations de statut ;
la prothèse (700) est configurée pour réagir à la commande suivante dans au moins le second état d'entrée : entrée pour quitter le second état d'entrée ; et
la prothèse (700) est configurée pour quitter automatiquement le second état de réception d'entrée après une séquence d'inaction au cours d'une période ne dépassant pas 20 secondes.

5. Dispositif selon l'une quelconque des revendications 1 à 4, dans lequel :
la prothèse (700) est configurée pour passer dans le second état de réception d'entrée uniquement en cas d'entrée d'une entrée codée complexe pendant le premier état de réception d'entrée.

6. Dispositif selon l'une quelconque des revendications 1 à 5, dans lequel :
la prothèse (700) est configurée pour recevoir une entrée sur la base de commandes d'entrée tactiles quand elle se trouve dans le premier état de réception d'entrée ; et
la prothèse (700) est configurée pour analyser l'entrée reçue et déterminer, sur la base de l'analyse, si l'entrée indique une commande d'origine humaine visant à passer dans le second état de réception d'entrée, et passer dans le second état de réception d'entrée lorsqu'il est déterminé que l'entrée indique une telle commande.

7. Dispositif selon l'une quelconque des revendications 1 à 6, dans lequel :
la prothèse (700) est une prothèse totalement implantable ; et
les commandes d'entrée tactiles proviennent d'un humain qui touche de la peau à proximité d'un capteur implanté de la prothèse (700).

8. Dispositif selon l'une quelconque des revendications 1 à 7, dans lequel l'entrée est basée sur une entrée tactile, et la prothèse (700) est configurée pour évaluer l'entrée afin d'identifier une commande provenant d'un bénéficiaire de la prothèse (700).

9. Procédé selon la revendication 8, dans lequel :
la prothèse (700) est configurée avec au moins l'un de sous-composants électroniques ou d'un micrologiciel/logiciel qui réduit une évaluation faussement positive de l'entrée par rapport à ce qui aurait été le cas en l'absence d'une telle configuration de la prothèse (700).

10. Procédé selon la revendication 8 ou la revendication 9, dans lequel :
l'entrée tactile sur laquelle est basée l'entrée et à laquelle la prothèse (700) est configurée pour réagir comporte au moins deux entrées parmi une entrée par tapotement, une entrée par glissement, une entrée de type cercle ou une entrée par maintien ;
la prothèse (700) est configurée de façon à réagir différemment à une première entrée parmi les au moins deux entrées par tapotement, par glissement, de type cercle ou par maintien, par rapport à une seconde entrée parmi les au moins deux entrées par tapotement, par glissement, de type cercle ou par maintien, et vice versa.

11. Dispositif selon l'une quelconque des revendications 8 à 10, dans lequel :
la prothèse (700) est une prothèse auditive totalement implantable :
le capteur est un microphone implantable (212) ;
la prothèse (700) comporte également un accéléromètre implantable (270) ;
la prothèse (700) est configurée pour évaluer un premier signal provenant du microphone implantable (212) et réaliser au moins l'une parmi les actions suivantes : évaluer un second signal provenant de l'accéléromètre (270) ou prendre en compte l'absence du second signal provenant de l'accéléromètre (270) pour identifier que l'entrée est une commande.

12. Dispositif selon l'une quelconque des revendications 8 à 10, dans lequel :
la prothèse (700) est une prothèse auditive totalement implantable ;
le capteur est un microphone implantable (212) ;
la prothèse (700) ne comporte pas d'accéléromètre (270).

13. Dispositif selon l'une quelconque des revendications 8 à 12, dans lequel :
la prothèse (700) est une prothèse auditive équipée d'un système de suppression du bruit ; et
la prothèse (700) est configurée pour utiliser des composants du système de suppression du bruit d'une manière sensiblement différente de celle employée pour la suppression du bruit afin d'évaluer l'entrée et d'augmenter la probabilité d'une détection correcte de l'entrée par rapport à ce qui serait le cas sans une telle configuration.

14. Dispositif selon l'une quelconque des revendications 8 à 13, dans lequel :
la prothèse (700) est une prothèse auditive configurée pour distinguer des événements tactiles et des événements vocaux propres de manière significative sur le plan statistique et pour distinguer des événements tactiles et des événements sonores externes de façon significative sur le plan statistique ; et/ou
la prothèse (700) est configurée avec au moins l'un parmi des sous-composants électroniques ou un micrologiciel/logiciel qui recherchent une entrée tactile comprenant un ordre suivi d'un ordre inverse afin d'identifier une commande provenant d'un bénéficiaire de la prothèse (700).

15. Dispositif selon l'une quelconque des revendications 1 à 14, dans lequel le mécanisme de protection du second état de réception réduit d'au moins 50 % la probabilité de passer accidentellement dans le second état de réception d'entrée par rapport à ce qui serait le cas sans le mécanisme de protection du second état de réception.
